# EUROPEAN PATENT SPECIFICATION

(11) **EP 3 187 654 B1**
(45) Date of publication and mention of the grant of the patent: **05.05.2021**
(21) Application number: 15203186.0
(22) Date of filing: 30.12.2015
(51) Int. Cl.: D06M 16/00, D06M 15/11, D06M 15/643, D06M 15/53, D06M 15/507, D06M 11/83, D06M 15/356, D06M 15/03, D06M 13/352, A01N 25/10, A01N 25/34, A01N 43/16, A01N 55/00, A01N 59/16, A61F 13/00, A61F 13/15, A61L 15/18, A61L 15/26, A61L 15/28, A61L 15/46, A61L 15/50, A61F 13/84

(54) **WASH-DURABLE ANTIMICROBIAL TEXTILE MATERIAL HAVING STAIN-RELEASE CAPABILITIES, IN PARTICULAR FOR REUSABLE SANITARY NAPKIN**
WASCHBESTÄNDIGE ANTIMIKROBIELLE TEXTILMATERIALEN MIT FLECKSCHUTZFÄHIGKEITEN, INSBESONDERE FÜR WIEDERVERWENDBARE DAMENBINDE
MATÉRIAU TEXTILE ANTIMICROBIEN PRÉSENTANT DES PROPRIÉTÉS DE RÉSISTANCE DES CAPACITÉS ANTI-TACHES, EN PARTICULIER POUR SERVIETTE HYGIÉNIQUE RÉUTILISABLE

(43) Date of publication of application: 05.07.2017
(73) Proprietor: Livinguard AG, 6300 Zug (CH)
(72) Inventor: SWAMY, Sanjeev, 6442 Gersau (CH); KURIEN, Ashok, Mumbai 400006 (IN)
(74) Representative: Karl, Christof

(56) References cited:
- WO-A1-2015/028852
- CH-A2- 706 466
- US-A- 5 747 392
- US-A- 5 993 840
- US-A1- 2005 062 010

## Description

### FIELD OF THE INVENTION

The present invention relates to a textile material having stain release, antimicrobial, and absorbing properties, and being wash durable. The textile material can be used, e.g., in a reusable sanitary napkin. The invention further relates to a method of finishing a textile material by applying and binding specific chemicals to a textile material so that the finished textile material exhibits the stain release, antimicrobial and absorbing properties such in a wash durable manner.

### BACKGROUND OF THE INVENTION

Disinfection is an important process in everyday life, in particular in the hygiene field, such as in female hygiene. Disposable sanitary napkins are well-known and often used. However, they produce great amounts of waste. Furthermore, the price of disposable sanitary napkins at a minimum of $ 3.50_{/}month is too high in particular for women in developing countries. For example, in 2015, two thirds of Indian women (about 400 million) cannot afford them. Instead of sanitary napkins, they oftentimes use pieces of old cotton clothes which are folded into a pad. However, use of such alternative sanitary care measures make women susceptible to yeast and fungal infections and they also run a higher risk of cervical cancer, reproductive tract and urinary tract diseases. It is desirable to overcome the waste problem and further to provide products for female hygiene, e.g. sanitary napkins, which prevent microbiological contamination.

Reusable sanitary napkins or sanitary towels are known in the art which have some disinfecting or antibacterial properties. For example, it is known to make sanitary napkins antibacterial by using tea (CN 201283041 Y), tetraammonium salt (CN 106559 A), or hexachlorophene (US 3,732,867 A). EP 2 829 256 A1 relates to a sanitary napkin comprising a contact layer (to the skin) being permeable to fluid passage, a pad for absorbing fluids, and an outer layer being impermeable to fluid passage. The pad comprises a percentage of antimicrobial fibers made from polymer material with silver particles added. However, all these solutions have disadvantages in that either the antimicrobial efficacy is low and/or the textiles are expensive to manufacture because of the ingredients used and/or because the ingredients are applied to the fiber or the yarn, not to the fabric. Furthermore, none of the prior art reusable napkins addresses the problem of staining in an efficient manner, so that in general a full wash cycle in a laundering machine is required before they can be reused.

From US 3,489,149 A, reusable sanitary napkins built into underwear are known, which use stain resistant and washable textiles. Furthermore, water-absorbing pads e.g. for diapers, are known from US 2001/0034510 A1. The water-absorbing pads are washable and reusable, wherein for washing an aqueous electrolytic solution (water and salts, e.g. magnesium hydroxide, sodium hypochlorite, and detergent) is used. However, none of these products exhibits antimicrobial activity.

Wash-durable textiles having antimicrobial properties and which are easily washable are desirable for a variety of further applications. One of their advantages is that the wash cycles and the resources required for laundering, such as energy, water, and detergent, can be reduced. This is particularly true for textiles which are used in hospitals or kitchens, as these textiles have a high exposure to staining materials like blood or food, and it is important that these textiles are not contaminated by microbes when they are worn or otherwise used. Water or oil repellent textile finishes can help to avoid staining, but some of these textiles, like kitchen towels or bed pads need to have good fluid absorbing capabilities, and therefor they should not be water or oil repellent.

US 8 906 115 B2 is directed to a method for antimicrobial finishing synthetic fibers or textiles, in which an aqueous solution of an organic primer component, an organic quaternary ammonium compound and a metal salt component is applied to the fibers. US5747392A discloses fabrics treated with two steps, first step comprises a biocide, second step comprises a stain resistant agent. The obtained textile is stain and mildew resistant.

WO2015/028852A1 discloses textiles rendered antimicrobial (such as for diapers) by an exhaustion process of a composition comprising antimicrobials, the textile is further treated to enable the bonding of active components (absorbent treatment for a diaper). Stain release is also cited as a property that can be imparted to the textile.

US2005/062010A1 discloses textiles having stain release and antimicrobial properties. The treatment composition comprising the antimicrobial and the stain release agent is applied in one step.

US5993840A discloses cellulosic non-woven materials treated with polymeric biguanide (PHMB) having antimicrobial properties. In one example, super absorbent is added to obtain an absorbent product for diapers. CH70666A2 discloses washable reusable sanitary napkins, the document is not concerned with any antimicrobial or stain release treatment.

### SUMMARY OF THE INVENTION

It is an object of the invention to provide textile materials that overcome some or all problems of any or all of above-mentioned prior art documents. It is a further object of the invention to provide textile materials exhibiting antibacterial properties even after numerous washes. Furthermore, it is an object of the invention that the textile materials can prohibit growth of bacteria, smells, odors etc. as completely as possible. It is another object that the textile materials are easily washable. It is a further object of the invention that the textile materials have fluid absorbing properties. It is another object that the textile materials exhibit properties rendering them suitable as hygiene products, e.g. as sanitary napkins.

Some or all of these objects are solved by the subject matter of the independent claims. Preferred embodiments are subject of the dependent claims.

In accordance with the present invention, a textile material is provided to which one or more chemical agents are fixed, which chemical agents convey antimicrobial, stain release properties and absorbing and/or adsorbing properties to the textile material.

The textile material can be used for hygiene products, e.g. sanitary napkins or lungotes. The invention further provides finishing methods for textile materials applying in particular padding and exhaust processes including heat treatments.

Without being bound to any theory, it is believed that reaction mechanisms or possible reaction products described hereinafter show which reactions can take place. However, the invention is in no way restricted to any reaction mechanism or possible reaction products described. These are provided for purposes of explanation only.

All percentages hereinafter refer to weight unless otherwise indicated.

The term "antimicrobial" as used in the context of the present invention relates to the ability to kill at least some types of microorganisms, or to inhibit the growth or reproduction of at least some types of microorganisms. Said term relates to any compound, agent, product or process that is harmful to one or more "microorganism" as used in the context of the present invention. Preferably, the one or more "microorganism" gets killed by the "antimicrobial" product or process.

The terms "microorganism" and "microbe", which are used interchangeably in the context of the present invention, are defined to comprise any organism too small to be seen by the unaided eye, such as, especially, single-celled organisms. In particular, the terms "microorganism" and "microbe" cover prokaryotes including bacteria and archaea, eukaryotes including protists, animals like dust mites or spider mites, fungi, and plants like green algae, as well as viruses.

The term "absorbing" commonly relates to the attachment or adhering of liquid at or near the surface of a textile material. The term "absorbing" commonly implies attachment or adhering of liquid in the interior of a textile material. The term "absorbing properties" as used herein means absorbing and/or adsorbing properties. Thus, the term "absorbing" or "absorbing properties" as used herein includes the attachment or adhering of liquid at or near the surface of a textile material and/or in the interior of a textile material. When the term "adsorbing" or "adsorbing properties" is used herein, it means the attachment or adhering of liquid at or near the surface of a textile material.

### DRAWINGS

In the following, preferred embodiments of the invention are described with reference to the figures, in which:
Figure 1 is a flowchart illustrating a process of manufacturing a textile material according to one embodiment of the invention.
Figure 2 a further flowchart illustrating a process of manufacturing a textile material according to another one embodiment of the invention.
Figure 3 shows a pad according to one embodiment of the invention.
Figure 4 shows the back side of the pad of Fig. 3.
Figure 5 shows the front side of the pad of Fig. 3.
Figure 6 shows a lungote or langote with inbuilt pad according to one embodiment of the invention.
Figure 7 shows a lungote or langote with inbuilt pad according to another embodiment of the invention.
Figure 8 shows a lungote or langote with inbuilt pad according to a further embodiment of the invention.
Figure 9 shows a lungote or langote without pad according to one embodiment of the invention.
Figure 10 shows a cross section of one embodiment of the invention illustrating the arrangement of the layers.

### DETAILED DESCRIPTION OF PREFERRED EMBODIMENTS

The textile materials, hygiene products, sanitary napkins, pads, lungot(e)s and/or langot(e)s can be produced using a process as described hereinafter.

### Process

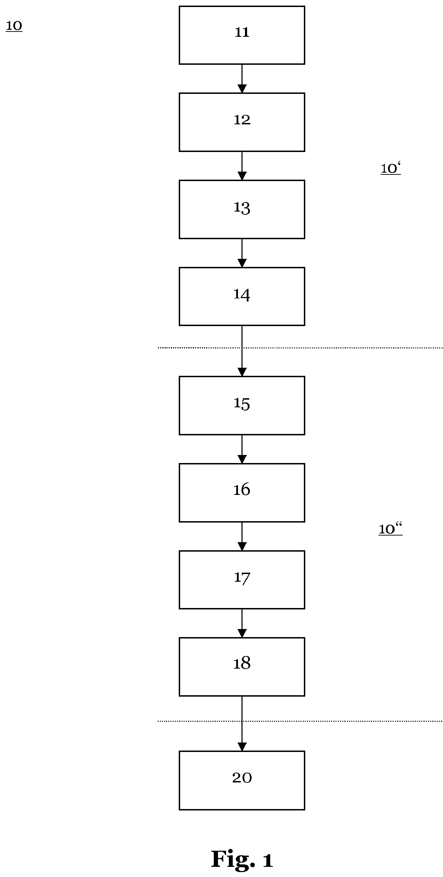

Fig. 1 shows the steps of a process 10 of finishing a textile material according to one embodiment of the present invention. In general, any textile material can be processed with said process 10. Preferably, the textile material is a synthetic textile material comprising or consisting of one or more selected from the following list: Rayon, nylon, non-acrylic olefin, acrylic polyester, PTFE, PP, PPE, carbon fiber, vinyon, saran, spandex, vinalon, aramids, modal, sulfar, polybenzimidazole fibre, PLA, lyocell, orlon, vectran and zylon acrylonitrile.

Preferably, the textile material is a natural textile material comprising one or more selected from the following list: Wool, cotton, cellulose, silk, linen, viscose, hemp, ramie and jute. Preferably, the textile material is a blend of a natural and synthetic textile material, in particular cotton, polyester, or a blend of cotton and polyester, preferably polyester. In another embodiment, the textile material comprises or consists of polyester. In a further embodiment, the textile material is a fabric of biodegradable material, preferably poly lactic acid (PLA). Preferably, the textile material comprises a stretch yarn, in particular lycra or spandex. Preferably, the textile material comprises or consists of terry fleece, which is in particular brushed or raised. The skilled person understands that several of the components listed above may be combined and thereby form the textile material to be finished with process 10.

The overall process 10 or preprocess 10 of Fig. 1 can be divided into two process cycles, a first antimicrobial process cycle 10' and an optional second antimicrobial process cycle 10". The first antimicrobial process cycle 10' may comprise the steps 11-14, while the second antimicrobial process cycle 10" may comprise the steps 15-18. The skilled person understands that also the first antimicrobial process cycle 10' may be optional in certain embodiments. The first antimicrobial process cycle 10' starts with an exhaust process 11. Although an exhaust process is performed during the first antimicrobial process cycle 10', a padding process might be performed instead in certain embodiments. As is known in the art, during an exhaust process, a textile material is guided through a container filled with a liquor, which liquor comprises ingredients which are transferred to the article during the exhaust process. Yarn and fabrics are typically treated with exhaust processes. During a common exhaust process, chemicals to be applied to a textile material are dissolved, according to the required liquor ratio, optionally with an auxiliary solution. Following, the textile material is immersed into the liquor, whereby the chemicals preferably contact the fibers and more preferably enter the fibers. For maintaining proper diffusion and penetration of the chemicals or molecules, a respective temperature and respective exhaustion time are set, such that kinetic and thermodynamic reactions take place as desired. As the textile material and its fibers absorb the chemicals, the concentration thereof in the liquor decreases. As is known in the art, the degree of liquor exhaustion as a function of elapsed time is termed rate or extend of the exhaust process. According to the present invention, the liquor of the exhaust process of the first antimicrobial process cycle comprises at least one chemical agent which increases the antimicrobial efficacy of the textile material. A detailed description of the liquor and of a textile material's antimicrobial properties and the antimicrobial efficacy of the textile material are given elsewhere in the description. Preferably, the exhaust process 11 is performed in an ambience with an ambient temperature higher than room temperature.

Generally, one or more chemical agents of the exhaust liquor, which increases the antimicrobial efficacy of the textile material, are preferably selected from the group consisting of quaternary ammonium organosilane compounds, silver cations, polyglucosamine, propiconazole, polyhexamethylene biguanide, and preferably of quaternary ammonium organosilane compounds, silver cations, propiconazole and/or carbendazim and/or isothiazoline. Preferably, the liquor of exhaust process 11 comprises silver cations trapped in an inorganic or organic matrix, preferably in an amount of 0.1 to 15% by weight, more preferably of 0.1 to 10% by weight, more preferably in an amount of 0.1 to 5% by weight, based on the weight of the textile material. Preferably, polyglucosamine is used in an amount of 0.1 to 5% by weight, preferably in an amount of 0.2 to 3% by weight, based on the weight of the textile material. Preferably, polyhexamethylene biguanide is used in an amount of 0.05 to 5% by weight, preferably in an amount of 0.1 to 3% by weight, based on the weight of the textile material. Preferably, propiconazole is used in an amount of 0.05 to 5% by weight, preferably in an amount of 0.1 to 3% by weight, based on the weight of the textile material. In a preferred embodiment, the liquor of exhaust process 11 contains the one or more antimicrobial agents in an amount of 0.1 to 20% by weight, in particular 0.1 to 15% by weight, preferably 0.1 to 10% by weight, more preferably 0.1 to 8% by weight, most preferably 0.1 to 5% by weight, based on weight of the textile material. Thereby an optimal performance of the first antimicrobial process cycle is achieved.

Preferably, the temperature of the liquor during the exhaust process is sufficiently high and the exhaust time is sufficiently long such that at least one chemical agent of the liquor is substantially uniformly dispersed across the cross section of the textile material as a result of the exhaust process. Thus, the temperature of the liquor should be sufficiently high and the exhaust time should be sufficiently long such that preferably the textile material is well impregnated and the chemical agent is dispersed throughout the entire textile material. Preferably, the exhaust time is sufficiently long and the temperature of the liquor during the exhaust process is sufficiently high such that the textile material can obtain desired antimicrobial properties, particularly after a respective heat treatment process, as will be outlined below.

It is generally preferred that the exhaust liquor has a temperature of at least 45 °C during the exhaust process 11. As known in the art, excessive heat may lead to yellowing of the textile material, which may be undesirable. Accordingly, the temperature of the liquor should not be too high. At too high temperatures, too much steam forms, reducing the efficiency of the process. Furthermore, if the temperature of the liquor is too high, turbulences can occur within the liquor bath and the textile material may get harmed. Further, with increasing exhaust time, the textile material may become weaker, i.e. its breaking strength may decrease.

The term exhaust time when used in the context of the present invention may preferably be defined as the period starting when at least part of the entire batch of textile material first comes into contact with the liquor and lasting until the batch is taken out of the exhaust equipment for e.g. advancing with a subsequent drying step.

The exhaust process 11 allows for evenly spreading the liquor across the entire cross section of the textile material, such that preferably no spot of the textile material is left untouched by the liquor. As a result, interactions and/or bonds may be created between the textile material and one or more chemical agents at this time.

The exhaust process 11 may be performed by any suitable technique, and is preferably performed in a jet-dyeing machine, a continuous dyeing range machine, or a jigger, and such that preferably most of the chemical agents of the exhaust liquor is exhausted evenly onto the entire cross section of the textile material. Preferably, an exhaustion rate of the exhaust process is at least 75%, more preferably at least 85%, more preferably at least 90%, and most preferably at least 95%, such that the textile material picks up most preferably about 95% of the chemical agents contained in the exhaust liquor, which increase the antimicrobial efficacy of the textile material. This exhaustion rate allows for reducing costs, as most of the ingredients of the liquor are exhausted by the textile material. It is also more ecological than processes with lower pickup rates.

In a preferred embodiment, the liquor of the exhaust process 11 has a temperature of at least 45 °C, more preferred of at least 50 °C, more preferably of at least 60 °C, more preferably of at least 70 °C, even more preferably at least 75 °C, and most preferably at least about 80 °C. Thus, it will be appreciated that the temperature of the liquor during the exhaust process 11 is sufficiently high. Preferably, during the exhaust process 11, the liquor has a temperature below boiling temperature, preferably of at most 95 °C, more preferably of at most 90 °C, more preferably of at most 85 °C, and most preferably at most about 80 °C. Thus, it will be appreciated that the temperature of the liquor during the exhaust process is sufficiently low. The preferred temperature of the liquor during the exhaust process 11 is about 80 °C, which provides particularly advantageous effects with regard to the antimicrobial properties of the textile material in the sense of the present invention.

Preferably, the exhaust time of exhaust process 11 of the first antimicrobial process cycle is at least 10 minutes, preferably at least 20 minutes, more preferably at least 30 minutes, more preferably at least 45 minutes, more preferably at least 50 minutes, more preferably at least 55 minutes, and most preferably at least about 60 minutes. Thus, it will be appreciated that the exhaust time is sufficiently long. Preferably, the exhaust time is at most 120 minutes, preferably at most 110 minutes, more preferably at most 100 minutes, more preferably at most 90 minutes, more preferably at most 80 minutes, more preferably at most 70 minutes, more preferably at most 65 minutes, and most preferably at most about 60 minutes. Thus, it will be appreciated that the exhaust time is sufficiently short. The preferred exhaust time is about 60 minutes, which provides particularly advantageous effects with regard to the antimicrobial properties of the textile material in the sense of the present invention.

The inventors found that the preferred temperature of the liquor during the exhaust process and the exhaust time is substantially independent of the weight of the textile material. Accordingly, different textile materials can easily be treated by means of the exhaust process 11 without having to change parameters of the exhaust process, while still obtaining the best possible results.

Preferably, during the exhaust process 11, the liquor is stirred. The stirring should be performed at intervals of less than 30 seconds, in other words, the stirring is performed regularly during the exhaust process 11 with interruptions of not more than 30 seconds. It will be appreciated that other suitable intervals may preferably be set, depending on the specific application. Ideally, the stirring is performed continuously during the exhaust process 11. This intermixing of the chemicals in the exhaust bath increases reliability of the exhaust process 11, as one or more chemical agents are more evenly distributed in the bath and as a result, a product with even quality throughout the entire textile material can be obtained. Preferably, the stirring is performed by means of a circulation pump, which circulates the liquor inside the exhaustion bath and which is typically comprised by a conventional exhaustion machine. In another embodiment, the stirring is performed by means of a stirrer which is inserted into the exhaustion bath. The stirrer may work at a speed of at least 200 rpm, more preferably at a speed of at least 250 rpm, and most preferably at a speed of at least 300 rpm. The stirrer used by the inventors is a simple mixer, which is similar to but larger than a standard household mixer. Preferably, the mixer has a minimum of three blades, which blades are preferably at least 10 cm long and preferably at least 2 cm wide. The stirrer was added by the inventors to the exhaustion machine they used as it is not provided by conventional exhaustion machines. Most preferably, the liquor is stirred by means of both a circulation pump and a stirrer. Due to this extensive mixing of the liquor, the exhaust process 11 is supported and one or more chemical agents are well dispersed across the cross section of the textile material during the exhaust process 11. As is known in the art, an exhaust process is typically applied for dyeing cloth, for example. In such applications, typically only a circulation pump is applied for ensuring proper fluid characteristics of the bath, such that a homogeneous dispersion of the dyeing molecules is present in the bath. However, since the chemical agents used in the context of the present invention can be less soluble in water compared to dyeing agents, the utilization of both a stirrer and a circulation pump assures that the chemical agents are not undissolved and do not settle at the bottom of the bath. Instead, due to the combination of both stirring means, the chemical agents are uniformly and homogeneous dispersed throughout the bath.

Accordingly, with exhaust process 11, the at least one chemical agent which increases the antimicrobial efficacy of the textile material, is substantially uniformly dispersed across the cross section of the textile material.

The exhaust process 11 is followed by a heat treatment which may start with drying process 12. The drying can be performed by using normal heat setting processes, depending on the actually used textile material. Preferably, the drying of the textile material is conducted at least partially at a temperature of at least 70°C, more preferably at least 100°C, more preferably at least 110°C, and most preferably at least 120°C. Preferably, the drying of the textile material is conducted at a temperature of at most 190°C, more preferably at most 180°C, more preferably at most 170°C, more preferably at most 160°C, more preferably at most 140°C, more preferably at most 130°C and most preferably at most 120°C. Most preferred, the drying temperature is about 120°C. Preferably, the drying time at the temperatures given above is of at least 10 seconds, preferably at least 20 seconds, and most preferably at least 30 seconds per 60 grams of textile material per square meter. Preferably, the drying time at the temperatures given above is of at most 100 seconds, more preferably at most 80 seconds, and most preferably at most 60 seconds per 60 grams of textile material per square meter. It will be appreciated that the drying times may increase with increasing weight of the textile material (per m²).

Drying process 12 may be conducted by passing the textile material through a stenter or stenter frame or similar drying machine. By drying the textile material, preferably excess moisture is removed.

After drying process 12, the heat treatment continues with curing process 13, as shown in Fig. 1. Any suitable machine can be utilized for performing the curing process 13, allowing for providing sufficient heat and sufficient dwell times. For example, a stenter may be used for the curing process 13.

Preferably, the curing temperature is sufficiently high and the curing time is sufficiently long such that one or more chemical agents of the liquor exhausted by the textile material are sufficiently strongly fixed or bonded to the textile material. They should preferably be set such that the chemical agents are bound to the textile material and optionally polymerized, become an inherent part of the textile material and provide the desired antimicrobial properties of the textile material. Depending on the agents and chemicals used, also crosslinking of the chemical agents may take place during the curing step. As a result thereof, the resultant textile material can favorably withstand several washes without losing its antimicrobial properties. The curing time depends on the type and weight of the textile material. However, the inventors found that the preferred curing temperature, which will be detailed below, is substantially independent of the weight of the textile material.

Preferably, the temperature of the liquor during the exhaust process is sufficiently high and the exhaust process is sufficiently long and the curing temperature is sufficiently high and the curing time is sufficiently long such that after washing the textile material, the favorable antimicrobial properties can be achieved. A washing of the resulting textile material may be done with water, preferably in a bath using warm to hot water in order to remove any residual chemicals for about an hour. Preferably, the water has a temperature in the range of 20°C and 60°C, and the washing is preferably performed between 30 minutes and 90 minutes, and further preferably is in accordance with the washing procedure outlined below.

Preferably, the curing temperature is sufficiently low and the curing time is sufficiently short such that the textile material does not discolor and/or turn yellow, and/or its breaking strength is not significantly reduced. Further preferred, the curing temperature is sufficiently low and the curing time is sufficiently short such that the textile material does not melt and/or burn and/or yellow, and/or that the colors of the textile material do not substantially change (discolor) as a result of the curing. Preferably, the temperature of the liquor during the exhaust process and the exhaust time and the curing temperature are such that the above favorable characteristics are achieved. In the most preferred embodiment, the temperature of the liquor during the exhaust process is 80 °C, the exhaust time is 60 minutes, and the curing temperature is 180 °C, which values are preferably independent from the textile material treated with process 10.

Thus, curing process 13 is preferably conducted at least partially at a curing temperature of at least 150°C, preferably at least 160°C, more preferably at least 170°C, even more preferably at least 175°C, and most preferably at least about 180°C. Preferably, curing process 13 is conducted at a temperature of at most 200°C, preferably at most 195°C, more preferably at most 190°C, even more preferably at most 185°C, and most preferably at most about 180°C. Thus, the preferred curing temperature is about 180°C.

Preferably, curing process 13 is performed at the temperature discussed above over a period (curing dwell time) of at least 10 seconds, more preferably at least 20 seconds, and most preferably at least 30 seconds per 60 grams of textile material per square meter. Preferably, the time period during which this temperature is applied is at most 100 seconds, more preferably at most 80 seconds, and most preferably at most 60 seconds per 60 grams of textile material per square meter. Thus, in the most preferred embodiment, a curing temperature of about 180°C is applied for about 60 seconds per 60 grams of textile material per square meter.

Since the curing temperature is substantially independent from the textile material, only the curing time (and drying time) have to be adjusted when using different textile materials. The inventors found that the curing time, or dwell time, increases about linearly with increasing weight of the textile material.

Preferably, curing process 13 immediately follows drying process 12 of process 10 illustrated in Fig. 1. Thus, the textile material preferably does not substantially cool down between the drying process 12 and the curing process 13. Drying process 12 and curing process 13 may be performed in one pass by passing the textile material through a stenter if curing process 13 immediately follows drying process 12. The skilled person understands that generally a heat treatment process may be performed after the exhaust process 11. The heat treatment process may comprise drying process 12 and/or curing process 13.

Still referring to Fig. 1, the heat treatment comprising drying process 12 and/or curing process 13 is followed by a washing process 14. During washing process 14, the textile material is preferably washed in water, further preferably without using detergents. Preferably, the textile material is washed in a bath, such as e.g. a water bath, having a temperature between 30°C and 50°C, further preferably between 35°C and 45°C. The washing time is preferably at least 35 minutes and more preferably at least 40 minutes. Washing process 14 preferably further comprises the step of drying the textile material, which drying can preferably be performed by means of a stenter. Washing process 14 preferably removes any surface contamination resulting from exhaust process 11.

After the first antimicrobial process cycle 10', the resulting textile material already features improved antimicrobial properties, as the antimicrobial efficacy of the textile material is increased. However, the antimicrobial efficacy of the textile material can be further increased by conducting a second antimicrobial process cycle 10" following the first antimicrobial process cycle 10'. Thus, process 10 of Fig. 1 comprises a padding process 15 for treating the textile material. Other standard techniques for treating textiles can be used in the alternative, such as e.g. an exhaust process, coating process or spraying process. However, utilizing a padding process has proven to be particularly advantageous because it is less expensive than exhaustion, it provides for a more even distribution of the liquor than spraying, and it yields better results as the coating paste typically contains ingredients which tend to leak. The skilled person understands that the second antimicrobial process cycle 10" described herein may also be performed independent of whether the first antimicrobial process was performed or not. Thus, as the first antimicrobial process cycle 10' may be optional, the second antimicrobial process cycle 10" may be performed as the initial process of the overall process of finishing a textile material.

Any suitable technique can be utilized for performing padding process 15, in which preferably a respective liquor is prepared and fed through a pump to a respective padding mangle. Accordingly, padding process 15 preferably comprises applications of one or more rolls to obtain optimum wet pickup of the liquor on the textile material. The appropriate padding mangle pressure is typically predetermined, depending on the quality of the textile material, and it is in general set such that the wet pickup of the agents is optimized. Preferably, the liquor again comprises at least one chemical agent which increases the antimicrobial efficacy of the textile material. The liquor may be formed similar to the liquor of the exhaust process 11 described above. Preferably, the liquor of padding process 15 is the same liquor as the one of exhaust process 11 desribed above. In a preferred embodiment, the liquor of the padding process comprises, in addition to one or more of the chemical agents conveying antimicrobial properties as described above for the liquor of the exhaust process, carbendazim and/or an isothiazoline. Preferably, the carbendazim and isothiazoline together are used in an amount of 0.1 to 10% by weight, preferably in an amount of 0.1 to 5% or 0.1 to 3 % by weight, based on the weight of the textile material. The liquor of the padding process may be at room temperature or it may be heated during the padding process. Preferably the liquor has a temperature of at least 15°C during the padding process. More preferably, during the padding process, the liquor has a temperature of at least i8°C, more preferably at least 20°C, and most preferred at least 23°C. Preferably the liquor has a temperature of at most 50°C, more preferably at most 45°C, more preferably at most 40°C and most preferred at most 35°C. The treatment time of the padding process is preferably at least 0.1 second, more preferred at least 0.5 second, and most preferred at least 1 second. Preferably, the treatment time of the padding process is at most 5 seconds, more preferred at most 2 seconds, and most preferred at most 1 second. Preferably the padding process has a liquor pickup rate of at least 60%, more preferably of at least 70%, more preferably of at least 75% and most preferably of at least about 80%. Preferably the padding process has a liquor pickup rate of at most 130%, more preferably of at most 120%, more preferably of at most 110%, and most preferred of at most about 100%. The liquor pickup rate may describe the mass of padding liquor impregnated to the textile, per mass of textile to be impregnated.

With the second antimicrobial process cycle, after padding process 15, a heat treatment comprising the steps of drying 16 and curing 17 is performed, which are preferably the same as drying process 12 and curing process 13 described above. Again, as described above with regard to the first antimicrobial process cycle, the padding process 15 may be followed by a heat treatment, comprising the drying process 12 and/or curing process 13. Finally, a washing process 18 is preferably performed, which is typically the same as washing process 14 described above. Washing should remove any surface contamination resulting from padding process 15.

By performing said second process cycle comprising process steps 15, 16 and 17, the antimicrobial efficacy of the resulting textile material is increased, as now the textile material is covered more thoroughly by the at least one chemical agents. When only performing the exhaust process cycle of process steps 11-13, the textile material may undesirably feature spots which do not feature antimicrobial properties at all, or of less performance compared to other spots. By performing the second process cycle, these spots or holes are closed so that a product with even quality throughout the entire textile material can be obtained. Also when omitting the first antimicrobial process cycle 10', the second antimicrobial process cycle 10" increases the antimicrobial efficacy of the resulting textile material.

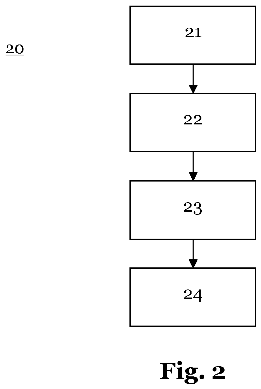

As illustrated in Fig. 1 the process 10 including the first and second antimicrobial process cycle is followed by a further process 20, comprising a main process cycle described in the following with reference to Fig. 2.

Fig. 2 shows the steps of a process 20 for finishing a textile material according to another embodiment of the present invention. With said process 20 in particular the stain release capability of the textile material may be increased. Also antimicrobial efficacy of the textile material may be increased with said process 20. As detailed above, in general any textile material may be processed with said process 20. However, preferably the textile material is a synthetic textile material, a natural textile material, a blend of a natural and synthetic textile material, or a fabric of biodegradable material, and preferably a textile material as described above, for example, with reference to the process 10 of Fig. 1. The process 20 may be performed after performing processes of the first and/or second antimicrobial processes described above. In particular embodiments, the preprocess 10 described above may be omitted.

The process 20 of Fig. 2 comprises the main process cycle according to the present invention. It preferably starts with a padding process 21. Generally any application process may be performed to apply a liquor to the textile which comprises one or more chemical agents which increase the antimicrobial efficacy and/or the stain release capability of the textile material. Thus, other standard techniques for treating textiles can be used in the alternative, such as e.g. an exhaust process, a knife or screen coating process or spraying process. If an exhaust process is applied, it may be performed in line with the process parameters detailed above, for example with the exhaust liquor having a temperature of 8o°C, and an exhaust process time of 60 minutes.. However, in a preferred embodiment, the padding technique is applied. Padding process 21 may be performed similar as described above with reference to process 10 of Fig. 1, for example with the padding liquor being at room temperature, and a liquor pickup rate of 80-100%. With process 20, the liquor of the padding process 21 comprises one or more chemical agents which increases the antimicrobial efficacy and/or the stain release capability of the textile material according to the present invention. A detailed description of the liquor and of a textile material's stain release capability are given elsewhere in the description. Preferably, the padding process 21 is performed in an ambience with an ambient temperature higher than room temperature, as detailed above.

Generally, the one or more chemical agents of the liquor of padding process 21, which agents increase the stain release capability of the textile material, are preferably based on at least one selected from the group consisting of carboxylic acid esters, polyester ether copolymers, organosilane terpolymers, and starch based emulsions. Preferably, the liquor comprises one or more chemical agents which increases the water holding capacity of the textile material. The water holding capacity of the textile material is detailed elsewhere in the description. Particularly preferred, a chemical agent which increases the water holding capacity of the textile material is based on organosilane terpolymers or starch based emulsion. This also applies if another application process is applied, such as e.g. a coating process.

The padding process 21 is followed by a heat treatment which may start with drying process 22. As detailed above, the drying can be performed by using normal heat setting processes, depending on the actually used textile material. Generally the description of drying processes 12 and 16 detailed above with respect to process 10 of Fig. 1 also applies here. With drying process 22, drying is preferably conducted at least partially at a temperature of at least 60 °C, preferably at least 100 °C, more preferably at least 110 °C, and most preferred at least 120 °C. Further, with drying process 22, drying is preferably conducted at least partially at a temperature of at most 190 °C, more preferably of at most 180 °C, and most preferably of at most 170 °C.

After drying process 22, the heat treatment continues with curing process 23, as shown in Fig. 2. As detailed above, any suitable machine can be utilized for performing the curing process 23, allowing for providing sufficient heat and sufficient dwell times. Generally the description of curing processes 13 and 17 detailed above with respect to process 10 of Fig. 1 also applies here. With curing process 23, the curing is conducted at least partially at a temperature of at least 160 °C, more preferred at least 170 °C, more preferably at least 175 °C, and most preferred at least 180 °C. Further, with curing process 23, curing is preferably conducted at a temperature of at most 200 °C, preferably at most 190 °C, more preferably at most 185 °C, and most preferably at most 180 °C. Preferably, the curing dwell time is at least 10 seconds, more preferably at least 20 seconds, and more preferably at least 30 seconds per 60 grams per square meter of fabric weight. Preferably the curing dwell time is at most 100 seconds, more preferably at most 80 seconds, and more preferably at most 60 seconds per 60 grams per square meter of fabric weight. The heat treatment of process 20 may comprise drying process 22 and/or curing process 23.

Curing process 23 is followed by a washing process 24. During washing process 24, the textile material is preferably washed in water, as detailed above. Generally the description of washing processes 14 and 18 detailed above with respect to process 10 of Fig. 1 also applies here.

The process 20 of Fig. 2 may follow one or more preceding antimicrobial process cycles, such as e.g. the first and/or second antimicrobial process cycle of the preprocess 10 described above with reference to Fig. 1. Thereby only single process steps of any one of the antimicrobial process cycles described above may be performed prior to the main process cycle. Also the main process cycle may comprise only part of the process 21-24 described above, for example only the padding process 21 and curing process 23. In an exemplary embodiment, the following steps may be performed: Antimicrobial process comprising exhaustion (e.g. at 80°C for 60 min) and drying (e.g. at 120°C for 30 or 120 seconds); Main process comprising padding and curing (e.g. at 180°C for 60 or 120 seconds). In another exemplary embodiment, the following steps may be performed: Antimicrobial process comprising padding and drying (e.g. at 120°C for 120 seconds); Main process comprising padding and curing (e.g. at 180°C for 120 seconds). In particular preferred embodiments, only one curing process is performed, namely following the application process of the main process cycle. Thus, the heat treatment of the optional antimicrobial process cycles preceding the main process cycle may comprise only a drying process, and the heat treatment of the main process cycle comprises a curing process.

It will be appreciated that one or more additional processes may be introduced between one or more of the individual processes of process 10 of Fig. 1 and/or process 20 of Fig. 2, and that some of the individual processes of process 10 of Fig. 1 and/or process 20 of Fig. 2 may be omitted. Furthermore, one or more additional processes may be performed prior or after performing the first and/or second antimicrobial process of process 10 of Fig. 1 and/or process 20 of Fig. 2. For example, before starting process 10 with the exhaust process 11 or padding process 21, the textile material should preferably be tested, washed and/or cleaned. Preferably, the fabric is first tested and if necessary washed or cleaned, so that the fabric is naturally hydrophilic in nature and free from all chemical contaminants that would hinder the application of the chemistry on the textile. Thereby, the fabric is advantageously freed from chemical contaminants that would hinder the application of later processes. In a particular preferred embodiment, one or more of the following steps may be performed prior to conducting process 10 of Fig. 1 and/or process 20 of Fig. 2: Testing the textile material at laboratory scale to verify and confirm that it meets respective selection criteria, batching and stitching together of individual textile pieces on a frame, inspecting the textile material thoroughly for defects, ensuring that the fabric is hydrophilic in nature and free from any chemical contaminants.

Preferably, the starting textile material is treated with a further antimicrobial agent, in particular one selected from the group consisting of benzalkonium chloride; benzethonium chloride; benzoxonium chloride; dequalinium; vinylbenzyltrimethylammonium chloride; cetrimonium bromide, optionally in combination with reactive amino silicone having hydroxyl groups or alkoxy groups such as methoxy or ethoxy groups; 2-phynolphenol, Acibenzolar, Paclobutrazol, Azoxystrobin, Epoxiconazole, Binapacryl, Iprodion, Triadimefon, Fuberidazole, Flusilazole, 2,4,6-tribromophenol, Vinclozolin, Pyrazophos, Tebuconazole, Metalaxy, Dichlofluanid, Strobilurins, Myclobutanil, Fenpropimorph with blocked isocyanate, vinylbenzyltrimethylammonium chloride, didecyldimethylammonium chloride, Fenticlor, 9-aminoacridine, Dibromopropamidine, Chlorothalonil, Povodine-Iodine, Fenamidone, Pencycuron, cetylpyridinium chloride, Cetrimonium, cetyl Trimethylammonium, Bupirimate, Fluopicolide, Hexachlorophene, Triclocarban, Nitrofura, Clioquinol, Methylparaben, Propamocarb, cinnamaldehyde, hexamidine, and Falcarindio. This may apply to any one of the antimicrobial processes of Fig. 1 and/or process 20 of Fig. 2. The further antimicrobial agent may be used in an amount of 0.1 to 10% by weight, preferably in an amount of 0.1 to 5% by weight, based on the weight of the textile material.

The textile material may be dyed prior to performing the process 10 or process 20. In another preferred embodiment, the textile material is finished to be multifunctional. After having performed process 10 or process 20, a respective multifunctional treatment may therefore be performed. With such a multifunctional treatment, the textile material may be provided with UV-blocking, water-repellent, mosquito-repellent and/or similar properties. It is also possible to conduct a multifunctional treatment in a padding process as described e.g. by padding process 15, wherein the padding liquor contains the respective functional agents. Preferably the liquor of process 11, process 15 and/or process 21 further comprises at least one functional agent selected from the group consisting of fluorocarbon chemicals, abrasion resistant agents, antistatic agents, anti-pilling agents, easy care resins, wetting agents, wicking chemicals, softeners, viscosity modifiers, fragrances, and pH modifiers. The functional agent may be used in an amount of 0.1 to 10% by weight, preferably in an amount of 0.1 to 5% by weight, based on the weight of the textile material.

### Stain release properties

The textile materials have stain release properties or stain release capabilities. The expressions "stain release properties" and "stain release capabilities" are used interchangeably herein. Stain release properties can be measured by using e.g. AATCC test method 130-2010. When measured using this test method, the rating of exemplary textile materials can be at least 3, in particular at least 4, preferably at least 5. The stain release properties can be imparted to the textile materials by finishing or treating a textile material with chemicals imparting or conveying stain release properties or capabilities. It is also possible to use a textile material which itself by nature or inherently, e.g. due to its starting material or polymer, possesses stain release properties. When using such a textile material, the stain release properties or capabilities can be increased or enhanced by using chemicals imparting or conveying stain release properties.

The textile materials have wash-durable stain release properties, In particular, the stain release properties, such as the AATCC ratings mentioned above, are achieved even after 25 washes in a laundry washing machine at 85±15 °C for 10-15 minutes using water and a detergent, e.g. using a non-antimicrobial, non-ionic and non-chlorine containing laundry detergent such as Tergitol 15-S-9 (from Dow Chemicals), preferably followed by a standard rinse cycle and preferably dried at 62-96 °C for 20-30 minutes.

Chemicals for conveying stain release properties to the textile materials are based on at least one selected from the group consisting of carboxylic acid esters, polyester ether copolymers, organosilane terpolymers, and starch based emulsions. In one embodiment, carboxylic acid esters are used. In another embodiment, polyester ether copolymers are used. In a further embodiment, organosilane terpolymers are used. Preferably, polyester ether copolymers and/or organosilane terpolymers are used. An exemplary embodiment of an organosilane terpolymer is Hydrosil, e.g. Hydrosil 8900, of Britacel Silicones. Due to the finishing process disclosed herein the chemicals are covalently bond, or strongly fixed, optionally by using an inbuilt binder, to the textile material such that they can withstand numerous washes. They do not leach off in washing or after several washings. It is also possible to use starch based emulsions, however, the fixing is weaker and can easier leach off than the other chemicals like carboxylic acid esters, polyester ether copolymers, or organosilane terpolymers.

Other exemplary embodiments of chemicals conveying stain release properties to textiles are Permalose of Croda Chemicals, or Feran ICA Base of Rudolf group based on polyester ether copolymers.

In particular embodiments, the chemicals conveying stain release properties are used in an amount of 0.05 to 5% by weight, in particular in an amount of 0.05 to 3% or 0.1 to 2% by weight, based on the weight of the textile.

### Antimicrobial properties

The textile materials preferably have antimicrobial properties or antimicrobial efficacy. The expressions "antimicrobial properties" and "antimicrobial efficacy" are used interchangeably herein. The antimicrobial properties can be imparted to the textile materials by finishing or treating a textile material with chemicals imparting or conveying antimicrobial properties or efficacy. It is also possible to use a textile material which itself by nature or inherently, e.g. due to its starting material or polymer, possesses antimicrobial properties.

The textile materials in particular exhibit increased antimicrobial efficacy against microorganisms which cause vaginal infections, such as fungal infections, bacterial infections, yeast infections, and/or viral infections. The textile material can exhibit antimicrobial efficacy against microorganisms which cause yeast infections, trichomoniasis, chlamydia, genital herpes, and/or genital warts. Specifically, the textile material may exhibit antimicrobial efficacy against Candida species, Lactobacillus species, including L. crispatus, L. gasseri, L. jensenii, L. iners; Gardnerella vaginalis, Mycoplasma hominis, Ureaplasma urealyticum, Peptostreptococcus. Mobiluncus, Prevotella, Bacteroides, Trichomoniasis vaginalis, Chlamydia trachomatis, Herpes simplex virus (HSV), and/or Human Papilloma Virus (HPV).

Antibacterial efficacy can be measured by using specific ATCC, ASTM or AATCC test methods. In exemplary embodiments, the textile material exhibits a reduction value of *Escherichia coli ATCC 25922* and/or *Staphylococcus aureus ATCC 6538* and/or *Pseudomonas aeruginosa ATCC 15442* and/or *Salmonella enterica ATCC 10708* and/or *Candida albicans* and/or *Aspergillus Niger,* measured in accordance with AATCC test method 100-2012 and/or ASTM E2149 - 10, of at least 90% (1 log), preferably at least 99% (2 log), more preferably at least 99.9% (3 log), within 1 hour of contact time, preferably remaining at the same level or greater even after 8 hours, more preferably increasing to at least 99.99% (4 log) after 8 hours.

The textile materials preferably have wash-durable antimicrobial properties. In particular, the antimicrobial properties are achieved even after e.g. 25 washes. In the textile material, the reduction values described above may be achieved even after at least 25 laundry washes in a laundry washing machine at 85±15 °C for 10-15 minutes using water and a detergent, preferably using a non-antimicrobial, non-ionic and non-chlorine containing laundry detergent, e.g. Tergitol 15-S-9 (Dow Chemicals), preferably followed by a standard rinse cycle and preferably dried at 62-96 °C for 20-30 minutes.

According to one embodiment of the invention, an antimicrobial agent or chemical agent increasing the antimicrobial efficacy is selected from a quaternary ammonium organosilane compound, silver cations, polyglucosamine (chitosan), propiconazole, and polyhexamethylene biguanide. In one embodiment a liquor used in the process described above comprises at least one of the antimicrobial agents selected from the group consisting of a quaternary ammonium organosilane compound, silver cations, polyglucosamine, propiconazole, and polyhexamethylene biguanide. In other embodiments, a liquor comprises at least two, at least three or at least four of the antimicrobial agents selected from the group consisting of a quaternary ammonium organosilane compound, silver cations, polyglucosamine, propiconazole, and polyhexamethylene biguanide. In further embodiments, a liquor comprises one, two, three or four of the antimicrobial agents selected from the group consisting of a quaternary ammonium organosilane compound, silver cations, polyglucosamine, propiconazole, and polyhexamethylene biguanide.

In further embodiments, the antimicrobial agent is carbendazim and/or an isothiazoline. This antimicrobial agent may be used alone or together with one, two, three, four or five antimicrobial agents selected from a quaternary ammonium organosilane compound, silver cations, polyglucosamine (chitosan), propiconazole, and polyhexamethylene biguanide. The use of carbendazim and/or an isothiazoline is preferably in a liquor for a padding process.

In another embodiment, a liquor comprises quaternary ammonium organosilane compound, silver cations, polyglucosamine, propiconazole, and polyhexamethylene biguanide. Such a combination of antimicrobial agents is particularly suitable, for example, for cotton or cellulosic materials as textile material.

In a further embodiment, a liquor comprises quaternary ammonium organosilane compound and silver cations; or chitosan and silver cations; or chitosan, carbendazim and an isothiazoline. Such combinations of antimicrobial agents are particularly suitable, for example, for polyester as textile material.

In a further embodiment, a liquor comprises chitosan, silver cations, polyhexamethylene biguanide, carbendazim and isothiazoline chitosan and silver cations. Such a combination of antimicrobial agents is particularly suitable, for example, for a blend of viscose and polyester as textile material.

An antimicrobial agent can comprise a quaternary ammonium organosilane compound. Suitable quaternary ammonium organosilane compounds have the formula 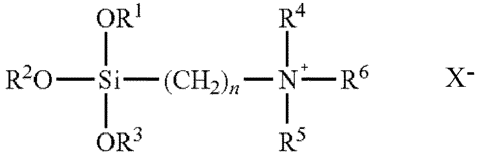 wherein the radicals have, independently of each other, the following meanings:
R¹, R², and R³ are a C₁-C₁₂-alkyl group, in particular a C₁-C₆-alkyl group, preferably a methyl group;
R⁴, and R⁵ are a C₁-C₁₈-alkyl group, a C₁-C₁₈-hydroxyalkyl group, a C₃-C₇-cycloalkyl group, a phenyl group, or a C₇-C₁₀-aralkyl group, in particular a C₁-C₁₈-alkyl group, preferably a methyl group;
R⁶ is a C-C₁₈-alkyl group, in particular a C₈-C₁₈-alkyl group;
X⁻ is the counterion and an anion, for example, chloride, bromide, fluoride, iodide, acetate, or a sulfonate group, preferably X⁻ is chloride or bromide; and
n is an integer of 1 to 6, in particular an integer of 1 to 4, preferably 3.

The term "alkyl group" as used herein means a branched or unbranched alkyl group.

Quaternary ammonium organosilane compounds are known in the art and commercially available. Such compounds possess specific functional groups which enable their bonding to functional groups of the textile material. Under the reaction conditions disclosed herein the quaternary ammonium organosilane compounds are bonded to the textile material via a covalent bond between the organosilane moiety and functional groups of the textile. Further, organosilane moieties polymerize with each other resulting in -O-Si-O- bonds. A possible reaction mechanism of the ammonium organosilane with a textile material having hydroxyl groups is shown hereinafter.

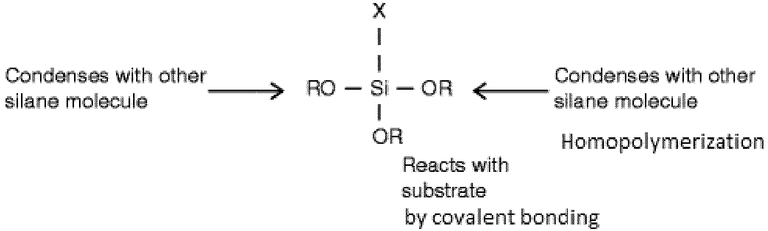

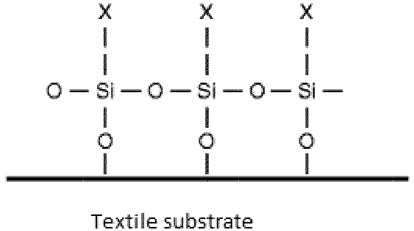

A possible reaction mechanism of the ammonium organosilane with silk having peptide groups (-CO-NH-) is shown hereinafter.

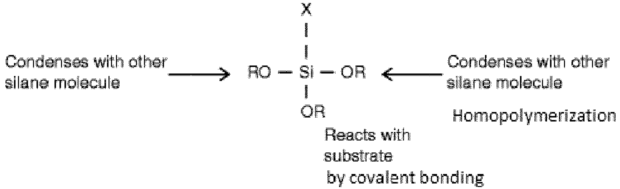

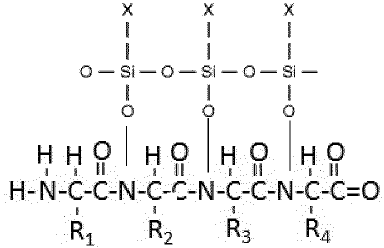

The quaternary ammonium organosilane compound can comprise dimethyloctadecyl[3-(trimethoxysilyl)propyl] ammonium chloride or dimethyltetradecyl[3-(trimethoxysilyl)propyl] ammonium chloride, most preferably dimethyloctadecyl[3-(trimethoxysilyl)propyl]ammonium chloride. The structure of dimethyloctadecyl[3-(trimethoxysilyl)propyl]ammonium is as follows (shown without counterion), wherein further the function of the silane moiety and the ammonium moiety are indicated:

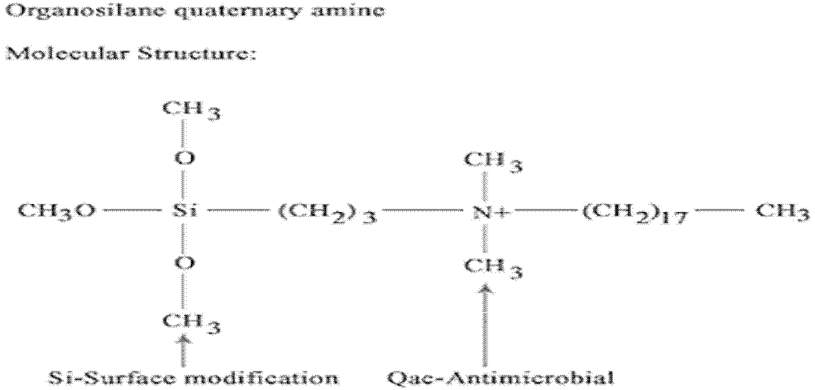

Dimethyloctadecyl[3-(trimethoxysilyl)propyl] ammonium chloride is available on the market, e.g. Aegis AEM 5772/5 (manufactured by Aegis). Dimethyltetradecyl[3-(trimethoxysilyl)propyl] ammonium chloride is available on the market, e.g. Sanitized T 99-19 (manufactured by Sanitized AG, Switzerland). Other suitable ammonium silane compounds are described, e.g., in patent applications US 2011/0271873 A1 and US 2006/0193816 A1, and in US patent 8,906,115. The quaternary ammonium organosilane compound is preferably used in an amount of 0.1 to 10% by weight, in particular in an amount of 0.1 to 5% or 0.1 to 3% by weight, based on the weight of the textile material.

An antimicrobial agent can comprise silver cations. In particular embodiments, the silver cations are trapped in an inorganic or organic matrix. Preferably, the inorganic matrix is an aluminosilicate. Preferably, the organic matrix is a polymeric matrix. Such silver-containing microbial agents are known in the art and available on the market.

A silver cation in form of its acrylate salt is shown hereinafter.

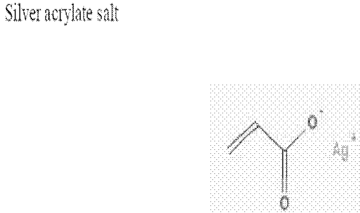

In an exemplary embodiment of the invention, the aluminosilicate is a sodium-poly(sialate-disiloxo) compound. Examples of an aluminosilicate and sialate structures as well as how bonding to a textile material can occur under the reaction conditions disclosed herein, are shown hereinafter.

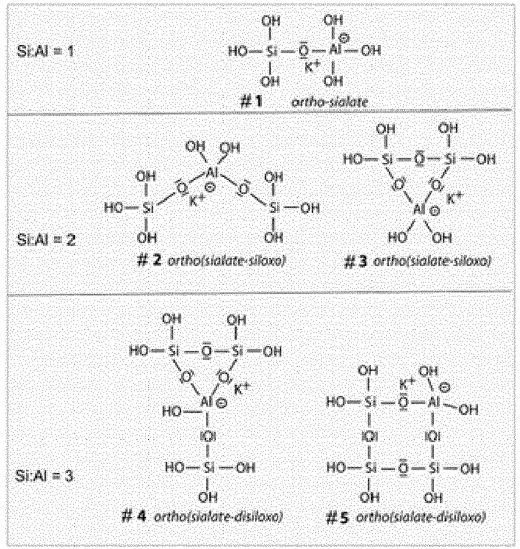

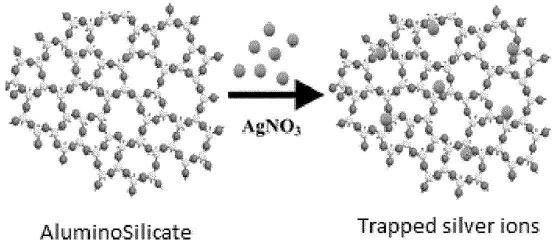

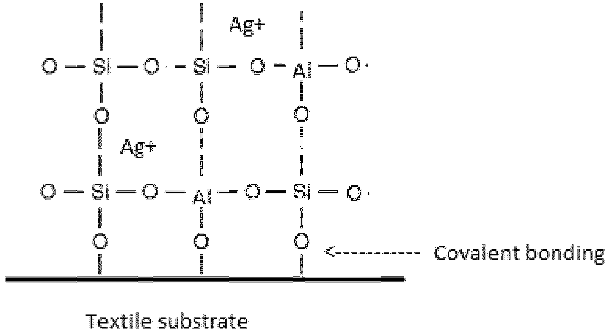

In an exemplary embodiment of the invention, the polymeric matrix into which silver cations are trapped is an acrylic polymer. Such silver-containing agents are known in the art and available on the market, e.g. SilvaDur AQ Antimicrobial (manufactured by Rohm and Haas) which contains acrylic polymer(s), silver nitrate, nitric acid and water. The silver cations trapped in an inorganic or organic matrix are preferably used in an amount of 0.1 to 15% by weight, preferably of 0.1 to 10% by weight, more preferably in an amount of 0.1 to 7% or 1 to 5% by weight, based on the weight of the textile material.

In another embodiment of the invention, an antimicrobial agent comprises polyglucosamine (chitosan). Chitosan has a structure as shown hereinafter, wherein n indicates the number of monomer units as known in the art:

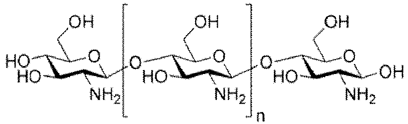

Under the reaction conditions disclosed herein, chitosan can react with -NH groups of silk resulting in covalent bonds as shown below.

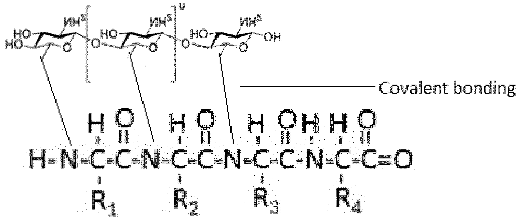

Under the reaction conditions disclosed herein, chitosan can react with functional groups of cellulosic materials resulting in covalent bonds as shown below.

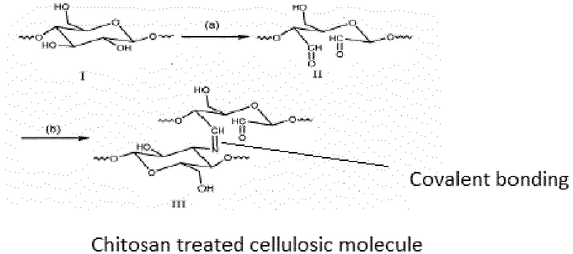

Chitosan is known in the art and commercially available. It is preferably used in an amount of 0.1 to 5% by weight, in particular in an amount of 0.2 to 3% or 0.2 to 2% by weight, based on the weight of the textile material.

In another embodiment of the invention, an antimicrobial agent comprises polyhexamethylene biguanide (PHMB). Polyhexamethylene biguanide has a structure as shown hereinafter, wherein n indicates the number of monomer units as known in the art.

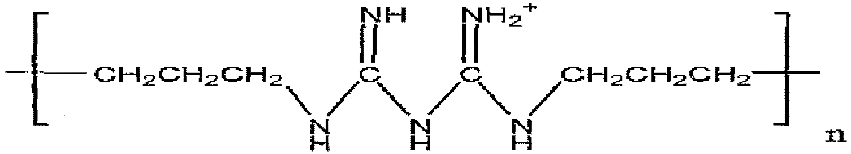

Under the reaction conditions disclosed herein, polyhexamethylene biguanide can react with hydroxyl groups of cellulose to form covalent bonds as shown hereinafter.

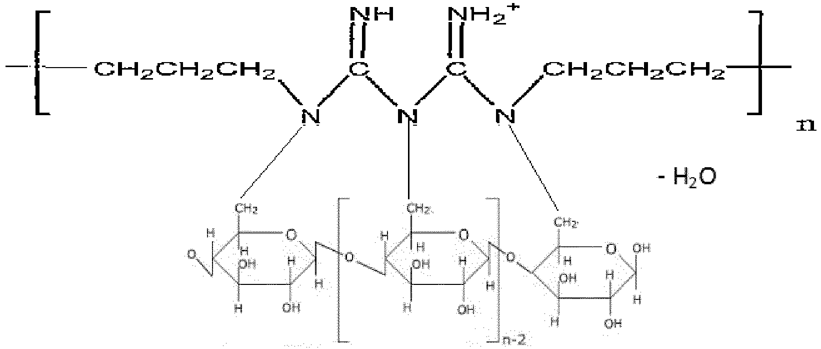

Under the reaction conditions disclosed herein, polyhexamethylene biguanide (PHMB) can react with carbonyl groups of silk fiber to form covalent bonds as shown hereinafter.

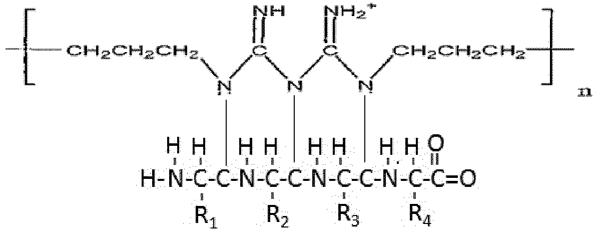

Polyhexamethylene biguanide is known in the art and commercially available. It can be used in an amount of 0.05 to 5% by weight, preferably in an amount of 0.1 to 3% or 0.2 to 2% by weight, based on the weight of the textile material.

In another embodiment of the invention, an antimicrobial agent comprises propiconazole. Propiconazole has a structure as shown hereinafter.

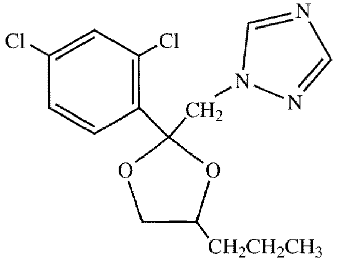

Propiconazole is known in the art and commercially available, e.g. Biogard PPZ 250 (manufactured by Beyond Surface Technologies, Switzerland). Propiconazole can be bound to the textile material using a crosslinking agent, in particular an isocyanate compound, which results in urethane bonds, or an acrylate based-product. When using propiconazole, it is preferred to use a crosslinking agent in the liquor, in particular the exhaust liquor. Further, it is preferred to use propiconazole together with an emulsifier. Propiconazole is preferably used in an amount of 0.05 to 5% by weight, in particular in an amount of 0.05 to 3% or 0.1 to 2% by weight, based on the weight of the textile material.

In another embodiment of the invention, an antimicrobial agent comprises carbendazim and/or an isothiazoline, in particular carbendazim and an isothiazoline or isothiazolinone. The isothiazoline or isothiazolinone may be e.g. 2-Octyl-2H-isothiazol-3-one. Carbendazim and isothiazolines are known in the art and commercially available, e.g. AFROTIN BF 5060 (manufactured by Schill and Seilacher, Germany). Carbendazim and/or an isothiazoline can be bound to the textile material using a crosslinking agent, e.g. an isocyanate compound, which results in urethane bonds, or an acrylate based-product. Preferably, an acrylate based-product, e.g. UKADAN 88 (manufactured by Schill and Seilacher, Germany), is used. When using carbendazim and an isothiazoline, it is preferred to use a crosslinking agent in the liquor, in particular the padding liquor. An antimicrobial agent comprising carbendazim and an isothiazoline is preferably used in an amount of 0.05 to 5% by weight, in particular in an amount of 0.05 to 3% or 0.1 to 2% by weight, based on the weight of the textile material.

In an exemplary embodiment, the antimicrobial agents comprise a quaternary ammonium organosilane compound and silver cations, preferably without polyhexamethylene biguanide and/or polyglucosamine and/or propiconazole. Another exemplary embodiment comprises the combination of a quaternary ammonium organosilane compound, polyhexamethylene biguanide and silver cations, wherein in particular no polyglucosamine and/or propiconazole is/are comprised. Such a combination is suitable for many textile materials, such as synthetic materials, cotton and cellulosic materials. Another exemplary embodiment provides for the combination of a quaternary ammonium organosilane compound, silver cations, polyhexamethylene biguanide, polyglucosamine, and preferably no propiconazole. Another exemplary embodiment provides for the combination of silver cations, polyhexamethylene biguanide and propiconazole, and no quaternary ammonium organosilane compound, preferably further no polyglucosamine.

In exemplary embodiments, a liquor used in the process described above contains the one or more antimicrobial agents in an amount of 0.1 to 20% by weight, in particular 0.2 to 15% by weight, preferably 0.5 to 10% by weight, more preferably 1 to 8% by weight, most preferably 1 to 5% by weight, or 0.03 to 4% by weight, based on weight of the textile material. Such amounts are particularly used in the liquor of the exhaust process or padding process.

In further embodiments of the invention, the starting textile material can be treated with one or more further antimicrobial agents, in particular at least one selected from the group consisting of benzalkonium chloride; benzethonium chloride; benzoxonium chloride; dequalinium; vinylbenzyltrimethylammonium chloride; cetrimonium bromide, optionally in combination with reactive amino silicone having alkoxy groups like hydroxyl or methoxy or ethoxy groups; 2-phynolphenol, Acibenzolar, Paclobutrazol, Azoxystrobin, Epoxiconazole, Binapacryl, Iprodion, Triadimefon, Fuberidazole, Flusilazole, 2,4,6-tribromophenol, Vinclozolin, Pyrazophos, Tebuconazole, Metalaxy, Dichlofluanid, Strobilurins, Myclobutanil, Fenpropimorph with blocked isocyanate, vinylbenzyltrimethylammonium chloride, didecyldimethylammonium chloride, Fenticlor, 9-aminoacridine, Dibromopropamidine, Chlorothalonil, Povodine-Iodine, Fenamidone, Pencycuron, cetylpyridinium chloride, Cetrimonium, cetyl Trimethylammonium, Bupirimate, Fluopicolide, Hexachlorophene, Triclocarban, Nitrofura, Clioquinol, methylparaben, Propamocarb, cinnamaldehyde, hexamidine, and Falcarindio. The further antimicrobial agent is preferably used in an amount of 0.1 to 10% by weight, in particular in an amount of 0.1 to 5%, 0.1 to 3% or 0.1 to 1% by weight, based on the weight of the textile material.

According to one embodiment (i) of the invention, an antimicrobial agent is selected from a quaternary ammonium organosilane compound, silver cations, polyglucosamine, propiconazole, and polyhexamethylene biguanide. In exemplary embodiments of embodiment (i), the textile material is treated with benzalkonium chloride, or benzethonium chloride, or benzoxonium chloride, or dequalinium or vinylbenzyltrimethylammonium chloride or cetrimonium bromide in combination with reactive amino silicone having hydroxyl or alkoxy groups, such as methoxy or ethoxy, instead of a quaternary ammonium organosilane. In exemplary embodiments of embodiment (i), the textile is treated with vinylbenzyltrimethylammonium chloride, or didecyldimethylammonium chloride, or Fenticlor, or 9-Aminoacridine, or Dibromopropamidine, or Chlorothalonil, instead of polyhexamethylene biguanide. In exemplary embodiments of embodiment (i), the textile is treated with Povodine-iodine, or Fenamidone, or Pencycuron, instead of polyhexamethylene biguanide and/or propiconazole. In exemplary embodiments of embodiment (i), the textile is treated with cetylpyridinium chloride, or Cetrimonium, cetyl trimethylammonium, or Bupirimate, instead of polyhexamethylene biguanide and/or quaternary ammonium organosilane. In exemplary embodiments of embodiment (i), the textile is treated with Fluopicolide instead of quaternary ammonium organosilane and polyhexamethylene biguanide or propiconazole. In exemplary embodiments of embodiment (i), the textile is treated with Nitrofura instead of quaternary ammonium organosilane. In exemplary embodiments of embodiment (i), the textile is treated with hexachlorophene, or Triclocarban, or Nitrofura, or Clioquinol, or Methylparaben, or Propamocarb, or Cinnamaldehyde, or Hexamidine, instead of chitosan and polyhexamethylene biguanide. In exemplary embodiments of embodiment (i), the textile is treated with Falcarindio in combination with polyhexamethylene biguanide or chitosan. In exemplary embodiments of embodiment (i), the textile is treated with 2-phynolphenol, or Acibenzolar, or Paclobutrazol, or Azoxystrobin, or Epoxiconazole, or Binapacryl, or Iprodion, or Triadimefon, or Fuberidazole, or Flusilazole, or 2,4,6-Tribromophenol, or Vinclozolin, or Pyrazophos, or Tebuconazole, or Metalaxy, or Dichlofluanid, or Strobilurins, or Myclobutanil, or Fenpropimorph, instead of propiconazole.

In preferred embodiments, the one or more chemical agents increasing the antimicrobial efficacy are substantially uniformly dispersed across the cross section of the textile.

### Water holding properties

The textile materials have water holding properties or water holding capacities. This means the ability of the textile material to absorb and/or adsorb water, in particular to absorb water. The expressions "water holding properties" and "water holding capacities" are used interchangeably herein. Water holding properties can be measured by using e.g. ASTM D7367-14. When measured using this test method, the water holding capacity of exemplary textile materials can be at least 3 times its weight, in particular at least 5 times its weight, preferably at least 7 times its weight, more preferred least 8 times its weight, and most preferred at least 12 times its weight. The water holding properties can be imparted to the textile materials by the textile material itself, i.e. by using a textile material which itself, e.g. due to its starting material, polymer or kind of fabric, possesses water holding properties. The water holding properties further can be imparted to the textile materials by finishing or treating a textile material with chemicals imparting or conveying water holding properties. It is also possible to combine both, i.e. to treat or finish a starting textile material exhibiting water holding properties with chemicals increasing the water holding capacity.

The textile materials preferably have wash-durable water holding properties. In particular, the water holding capacity is substantially unchanged after e.g. 25 washes.

The chemicals used for imparting or conveying water holding properties to the textile material can also absorb and/or adsorb liquids, such as water or aqueous media. In one embodiment, the chemical is based on an organosilane terpolymer. In a further embodiment, the chemical is based on polyester ether copolymers. In another embodiment, the chemical is based on carboxylic acid esters. It is believed that some chemicals impart at least partially adsorbing properties and partially absorbing properties. It is possible to use, as chemicals conveying water holding, absorbing and/or adsorbing properties, the same chemicals as the chemicals used for conveying stain release properties.

Exemplary embodiments of chemicals conveying water holding properties to textiles are Hydrosil of Britacel Silicones (Hydrosil is based on organosilane terpolymers); Permalose of Croda Chemicals; or Feran ICA Base of Rudolf group based on polyester ether copolymers.

In particular embodiments, the chemicals conveying water holding properties are used in an amount of 0.05 to 5% by weight, in particular in an amount of 0.05 to 7% or 0.1 to 5% by weight, based on the weight of the textile material.

### Further chemicals

In other embodiments of the invention, the liquor used in the process described above further comprises at least one functional agent selected from the group consisting of water and oil repellents, fluorocarbon chemicals, abrasion resistant agents, antistatic agents, anti-pilling agents, easy care resins, wetting agents, wicking chemicals, softeners, mosquito or insect repellants, UV protectors, soil releasing agents, viscosity modifiers, flame retardants, hydrophilic polymers, polyurethanes, fragrances, and pH modifiers. The functional agent, in particular a water- and oil-repellant, is preferably used in an amount of 0.1 to 10% by weight, in particular in an amount of 0.1 to 5%, 0.1 to 3% or 0.1 to 1% by weight, based on the weight of the textile material. The functional agent is preferably applied by a padding process, i.e. it is contained in the liquor of a padding process, but not in the liquor of an exhaust process. The textile material can be made multifunctional by adding the desired functional agent(s) along with or after the initial treatment, during the processing phase. In order to impart multifunctional capabilities, the textiles may be treated on one or both sides of the textile, either separately or jointly.

In preferred embodiments, a water- and oil-repellant is used. The water- and oil-repellant can be any repellant known in the art for this purpose. The water- and oil-repellant can contain polyurethane. Commercial products are e.g. Globe WP of Globechem Imports and Globe PUWP (with polyurethane) of Globechem Imports. The water- and oil-repellant can be applied by padding, exhaust or coating processes. Preferably, water- and oil-repellant without polyurethane are applied by padding process, and water- and oil-repellant with polyurethane are applied by a coating process. *[Correct?]* The water- and oil-repellants can be used in amounts of 50 to 100 gpl (grams per liter), preferably 60 to 90 gpl,-*?*] based on the liquor *[correct?].*

According to one embodiment (i) of the invention, an antimicrobial agent is selected from a quaternary ammonium organosilane compound, silver cations, polyglucosamine, propiconazole, and polyhexamethylene biguanide. In exemplary embodiments of embodiment (i), the textile material is treated with benzalkonium chloride, or benzethonium chloride, or benzoxonium chloride, or dequalinium or vinylbenzyltrimethylammonium chloride or cetrimonium bromide in combination with reactive amino silicone having hydroxyl or alkoxy groups, such as methoxy or ethoxy, instead of a quaternary ammonium organosilane. In exemplary embodiments of embodiment (i), the textile is treated with vinylbenzyltrimethylammonium chloride, or didecyldimethylammonium chloride, or Fenticlor, or 9-Aminoacridine, or Dibromopropamidine, or Chlorothalonil, instead of polyhexamethylene biguanide. In exemplary embodiments of embodiment (i), the textile is treated with Povodine-iodine, or Fenamidone, or Pencycuron, instead of polyhexamethylene biguanide and/or propiconazole. In exemplary embodiments of embodiment (i), the textile is treated with cetylpyridinium chloride, or Cetrimonium, cetyl trimethylammonium, or Bupirimate, instead of polyhexamethylene biguanide and/or quaternary ammonium organosilane. In exemplary embodiments of embodiment (i), the textile is treated with Fluopicolide instead of quaternary ammonium organosilane and polyhexamethylene biguanide or propiconazole. In exemplary embodiments of embodiment (i), the textile is treated with Nitrofura instead of quaternary ammonium organosilane. In exemplary embodiments of embodiment (i), the textile is treated with hexachlorophene, or Triclocarban, or Nitrofura, or Clioquinol, or Methylparaben, or Propamocarb, or Cinnamaldehyde, or Hexamidine, instead of chitosan and polyhexamethylene biguanide. In exemplary embodiments of embodiment (i), the textile is treated with Falcarindio in combination with polyhexamethylene biguanide or chitosan. In exemplary embodiments of embodiment (i), the textile is treated with 2-phynolphenol, or Acibenzolar, or Paclobutrazol, or Azoxystrobin, or Epoxiconazole, or Binapacryl, or Iprodion, or Triadimefon, or Fuberidazole, or Flusilazole, or 2,4,6-Tribromophenol, or Vinclozolin, or Pyrazophos, or Tebuconazole, or Metalaxy, or Dichlofluanid, or Strobilurins, or Myclobutanil, or Fenpropimorph, instead of propiconazole.

### Liquor

The following description relates to a liquor as it may be used in the process described above, in particular an exhaust or padding process.

According to a preferred embodiment, the liquor contains a solvent. The solvent is in particular water, but can contain other solvents being compatible with the other components of the liquor, e.g. methyl alcohol.

In one embodiment the liquor contains an emulsifying agent, in particular one selected from the group consisting of polyoxyethylene monostearate, polyoxyethylene sorbitan monolaurate, polyethylene glycol 400 monolaurate, ethylene oxide condensates, fatty alcohol ethoxylates, and sodium lauryl sulfates. The liquor can contain an emulsifying agent in an amount of 0.05 to 5% by weight, preferably of 0.1 to 2.5% by weight, based on weight of the textile material. Alternatively, the liquor can contain an emulsifying agent in an amount of 1 to 50 grams per liter of liquor, preferably of 1 to 25 grams per liter of liquor. Depending on the agents and chemicals used, an emulsifier can be used in the liquor of an exhaust process or liquor of a padding process, preferably it is used in an exhaust liquor. In other exemplary embodiments, the emulsifier is used in a concentration of between 5 mg to 100 mg per 100 grams of textile material weight, depending on the application.

In one embodiment of the invention, the liquor has a pH-value of at most 6.9, preferably at most 6.5, more preferably at most 6.3, in particular at most 6.0, most preferably at most 5.5. The liquor should have a pH-value of at least 3.0, preferably at least 3.5, more preferably at least 4.0, even more preferably at least 4.5, most preferably at least 5.0. The pH-value can be set or adjusted using an organic acid. Particularly suitable are citric acid, acetic acid, or a combination thereof, wherein preferably citric acid is used.

The liquor can contain chemicals conveying stain release properties, and/or water holding properties, and/or absorbing properties and/or antimicrobial properties. Chemicals conveying stain release properties are contained in the liquor in amounts of 5% to 10% based on weight of the textile material or 50g to 100g in 1 liter of the liquor. Chemicals conveying water holding and/or absorbing and/or, optionally adsorbing properties, are contained in the liquor in amounts of 5% to 10% based on weight of the textile material or 50g to 100g in 1 liter of the liquor. Chemicals conveying antimicrobial properties are contained in the liquor in amounts of 3% to 5% based on weight of the textile material or 30g to 50g in 1 liter of the liquor.

In exemplary embodiments of the invention, the liquor comprises a crosslinking agent which is selected from the type of adducts of blocked isocyanate, preferably in a concentration range of between 10mg to 200 mg per 100 grams of textile material weight, depending on application. Preferably in the same amounts, the liquor also may contain an acrylate based-product as crosslinking agent.

The finished textile material of the invention is, depending on the chemical(s) used for finishing, preferably hydrophobic or hydrophilic across its cross section. For example, a textile material finished with a water- and oil-repellant is hydrophobic. For example, a textile material finished with a polyester ether copolymer is hydrophilic.

In further embodiments of the invention, the liquor comprises at least one agent selected from the group consisting of methyl alcohol, octadecylaminomethyl trihydroxysilylpropyl ammonium chloride and chloropropyl trihydroxysilane, polyglucosamine, silver chloride based compound and silver chloride in aluminosilicate carrier base and polyhexamethylene biguanide, in a concentration range of between 10mg to 500mg of the active ingredient of each chemical per 100 grams of fabric weight, depending on application, and the polysaccharide or oligosaccharide in a concentration range of between 1mg to 500 mg of active ingredient per 100 grams of fabric weight, depending on the application.

### Textile material

Generally, any textile material can be used as the starting textile material. According to one embodiment of the invention, the starting textile material is hydrophilic, e.g. the textile material comprises hydrophilic groups. According to another one embodiment of the invention, the starting textile material comprises hydroxyl, peptide and/or carbonyl groups. These groups enable fixing, bonding, attaching or adhering of one or more of the chemicals conveying stain release properties, and/or of one or more of the chemicals conveying antimicrobial properties, and/or of one or more of the chemicals conveying water holding, absorbing and/or adsorbing properties, to the textile material. In exemplary embodiments, the starting textile material comprises peptide and/or hydroxyl groups, in particular hydroxyl groups. According to another embodiment of the invention, the textile material is a natural textile material, a synthetic textile material, or a blend thereof, in particular a blend of natural and synthetic textile material. Both natural and synthetic textile materials comprise functional groups having the ability to bond one or more of the chemicals to the textile material.

According to a specific embodiment of the invention the natural textile material comprises at least one material selected from the group consisting of wool, cotton, cellulose, viscose, silk, linen, hemp, ramie, jute, and combinations thereof. Preferred textile materials thereof are wool, cotton and/or silk, with cotton being especially preferred.

In a specific embodiment, the textile material comprises, essentially consists, or consists, of polyester. The polyester can be any polyester known in the art for textiles.

According to another specific embodiment of the invention, the synthetic textile material comprises at least one material selected from the group consisting of polyester, rayon, nylon, non-acrylic olefin, acrylic polyester, polytetrafluoroethylene (PTFE), polyethylene (PE), polypropylene (PP), polyphenylene ether (PPE), carbon fiber, vinyon, saran, spandex, vinalon, aramids, modal, sulfar, polybenzimidazole (PBI) fiber, polylactide (PLA), lyocell, polyacrylonitrile, orlon, vectran, zylon acrylonitrile, and combinations thereof. Preferred textile materials thereof are polyester and/or polyamide, in particular polyester. It is also possible to use, as synthetic textile material, polyamide or nylon.

According to a further specific embodiment of the invention, the textile material comprises cotton, polyester, or a blend of cotton and polyester. Preferably, the textile material comprises between 20% and 60% of cotton, more preferably between 25% and 50% of cotton, in particular between 30% and 40% of cotton. In particular, the textile material comprises between 40% and 80% of polyester, preferably between 50% and 75% of polyester, more preferably between 60 and 70% of polyester.

According to another specific embodiment of the invention, the textile material comprises viscose, or a blend of viscose and polyester. Preferably, the textile material comprises between 20% and 60% of viscose, more preferably between 25% and 50% of viscose, in particular between 30% and 40% of viscose. In particular, the textile material comprises between 40% and 80% of polyester, preferably between 50% and 75% of polyester, more preferably between 60 and 70% of polyester.

In another embodiment, the textile material comprises polylactide (PLA), consists essentially or consists of polylactide.

The term "textile material" as used herein means a textile material in any form and includes fibers, yarns, threads, ply yarns, fabrics produced from fibers and/or yarns, and the finished products produced from fibers, yarns, and/or fabrics. The textile material can be a fiber, a yarn, or a fabric, in particular a yarn or a fabric, in particular a fabric. The textile material can be woven, knitted, crocheted, bonded and/or non-woven fabric. The textile material can be selected from the group consisting of woven, knitted, crocheted, melt-blown, bonded, needle punched non-woven fabric, and polar terry fleece, loop fabric, either of one side or both sides. The textile material can be spun, electrospun, melt-blown, drawn or extruded. The textile material can be a mesh, a woven fabric, non-woven fabric, or terry fleece. In exemplary embodiments, the textile material comprises or consists of terry fleece, which optionally is brushed and/or raised.

The textile material may comprise between 20% and 70% of cellulosic fabric, preferably between 25% and 50% of cellulosic fabric, or alternatively preferably between 50% and 75% of cellulosic fabric. Alternatively, the textile material may comprise between 20 to 60% cellulosic fabric, preferably between 30 to 40% of cellulosic fabric. Alternatively, the textile material may comprise between 10 to 20% of cellulosic fabric. The cellulosic fabric can be cotton and/or viscose.

In other exemplary embodiments, the textile material comprises between 10% and 100% of synthetic fiber, preferably between 25% and 80%, more preferably between 50% and 75%, most preferably between 60% and 70%. The synthetic fiber can be e.g. polyester. Alternatively, the synthetic fiber can be e.g. polyamide or nylon.

In preferred embodiments, the starting textile material is hydrophilic in nature, clear of all auxiliaries and contaminants so that the liquor(s) can be applied to the textile without any hindrance or interference.

The textile materials obtained can be used, for example, as separate layers in a product for hygienic purposes, e.g. a sanitary napkin, wherein the separate layers have different functions. In exemplary embodiments, such products comprise an inner layer, an absorption layer, a water repellant layer, and optionally a transport layer.

### Inner layer (layer 1)

In a preferred embodiment of the invention, the textile material is used in an inner layer of a product or constitutes an inner layer. As used herein, inner layer means the layer closest to the body of the wearer. The inner layer serves to conduct any body fluids away from the body. The inner layer should remain dry, in order to keep the body of the wearer dry. Further, the inner layer should exhibit antimicrobial properties, in order to avoid infections.

In preferred embodiments, at least 95%, preferably at least 97%, more preferably at least 99%, of the textile material of the inner layer consists of a synthetic fiber, preferably polyester. It is also possible to use, as synthetic textile material, polyamide or nylon.

In preferred embodiments, the textile material of the inner layer is a mesh, is knitted, and/or a monofilament. In particular, the textile material has at least 9, preferably at least 16, more preferably at least 25, and most preferably at least 36 holes, per cm². The textile material can have at most 144, preferably at most 81, more preferably at most 64, and most preferably at most 49 holes, per cm². The size of the holes can be at least 0.05 mm, preferably at least 0.1 mm, more preferably at least 0.2 mm, and most preferably at least about 0.5 mm, in average diameter. Further, the size of the holes can be at most 2 mm, preferably at most 1 mm, more preferably at most about 0.5 mm, in average diameter.

In exemplary embodiments, the textile material has a weight of at least 25 GSM (grams per square meter), preferably at least 40 GSM, more preferably at least 50 GSM, and most preferably at least about 60 GSM. The textile material can have a weight of at most 125 GSM, preferably at most 100 GSM, more preferably at least 75 GSM, and most preferably at most about 60 GSM.

In preferred embodiments, the textile material can have one or more of the following features. The textile material can have a water absorbency time of at least 2 minutes, preferably at least 3 minutes, measured in accordance with AATCC test method 79-2014 (Option A). The textile material can have a water repellency rating of at least 70, preferably at least 80, more preferably at least 90, measured in accordance with AATCC test method 22-2014. The textile material can exhibit a stain release capability, measured in accordance with AATCC test method 130-2010, of at least rating 4, preferably rating 5. The textile material can exhibit a stain/oil repellency, measured in accordance with AATCC test method 118-2013, of at least rating 1, preferably at least rating 2, and more preferably at least rating 3, and most preferably at least rating 4. The textile material can exhibit a water holding capacity of at most 2.0 times its weight, preferably of at most 0.5 times its weight, more preferably at most 0.2 times its weight, when tested according to ASTM D7367 - 14. The textile material can exhibit a reduction value of *Escherichia coli ATCC 25922* and/or *Staphylococcus aureus ATCC 6538* and/or *Pseudomonas aeruginosa ATCC 15442* and/or *Salmonella enterica ATCC 10708* and/or *Candida albicans* and/or *Aspergillus Niger,* measured in accordance with AATCC test method 100-2012 and/or ASTM test method E2149 - 10, of at least 99% (2 log), preferably at least 99.9% (3 log), within 1 hour of contact time, preferably retaining the same performance or greater after 8 hours, more preferably increasing to at least 99.99% (4 log) after 8 hours. The reduction values can be achieved even after at least 25 laundry washes in a laundry washing machine at 85±15 °C for 10-15 minutes, preferably using a non-antimicrobial, non-ionic and non-chlorine containing laundry detergent, e.g. Tergitol 15-S-9 (Dow Chemicals), preferably followed by a standard rinse cycle and preferably dried at 62-96 °C for 20-30 minutes.

The textile material of the inner layer can be produced by the process described above. In the process, in particular the liquor applied in the main process cycle can contain polyglucosamine, preferably at least 2 gpl (grams per liter), more preferably at least 4 gpl, particularly at least 7 gpl, and most preferably at least about 10 gpl. The liquor applied in the main process cycle can contain an antimicrobial agent comprising carbendazim and isothiazoline, preferably at least 10 gpl (grams per liter), more preferably at least 25 gpl, particularly at least 40 gpl, and most preferably at least about 50 gpl. The liquor applied in the main process cycle may contain an acrylate-based cross linker, preferably at least 10 gpl (grams per liter), more preferably at least 25 gpl, particularly at least 40 gpl, and most preferably at least about 50 gpl.

The process for obtaining the textile material of the inner layer may comprise a curing dwell time of the main process cycle of at least 20 sec (seconds), preferably at least 40 sec, more preferably at least 50 sec, and most preferably at least about 60 sec, per 60 GSM of fabric weight. The process may further comprise a curing dwell time of the main process cycle of at most 120 sec (seconds), preferably at most 100 sec, more preferably at most 80 sec, and most preferably at most about 60 sec, per 60 GSM of fabric weight. In the process, an exhaust process can be used in the antimicrobial process cycle. Preferably, the drying dwell time of the antimicrobial process cycle is at least 10 sec (seconds), preferably at least 20 sec, more preferably at least 25 sec, and most preferably at least about 30 sec, per 60 GSM of fabric weight. Preferably, the drying dwell time of the antimicrobial process cycle is at most 60 sec, preferably at most 50 sec, more preferably at most 40 sec, and most preferably at most about 30 sec, per 60 GSM of fabric weight.

In preferred embodiments, the liquor applied in the antimicrobial process cycle contains at least 0.2%, preferably at least 0.4%, more preferably at least 0.6%, and most preferably at least about 0.8% of a quaternary ammonium organosilane compound, based on weight of fabric (owf). In addition or alternatively, the textile material preferably comprises at least 0.05%, preferably at least 0.1%, more preferably at least 0.15%, and most preferably at least about 0.2% of silver cations, based on weight fabric (owf).

When finishing the textile material with chemicals having antimicrobial properties, these chemicals additionally can provide at least partially or substantially hydrophobic character to the textile material at its surface and/or in its interior. This hydrophobic character can impart the textile material with stain release properties.

### Transportation layer (layer 2)

A transportation or transport layer is an optional layer in a product for hygienic purposes, e.g. a sanitary napkin. In a preferred embodiment of the invention, the textile material is used in a transportation layer or constitutes a transportation layer. As used herein, the transportation layer means the layer between the inner layer and the absorption layer. The transportation layer serves to conduct body fluids from the inner layer to the absorption layer.

The textile material of the transportation layer is preferably an impression fabric, preferably warp knitted, and preferably a multifilament. The textile material may comprise at least 20%, preferably at least 25%, more preferably at least 30%, most preferably at least about 35% viscose. The textile material may comprise at most 50%, preferably at most 45%, more preferably at most 40%, most preferably at most about 35% viscose. The textile material may comprise at least 50%, preferably at least 55%, more preferably at least 60%, most preferably at least about 65% of a synthetic fiber, such as nylon, polyamide, or preferably polyester. Preferably, the textile material comprises at most 80%, preferably at most 75%, more preferably at most 70%, most preferably at most about 65% of a synthetic fiber, such as nylon, polyamide, or preferably polyester. In other embodiments, at least 95%, preferably at least 97%, more preferably at least 99% of the textile material consists of a synthetic fiber, such as nylon, polyamide, or preferably polyester.

The textile material of the transportation layer can have at least 25, preferably at least 30, more preferably at least 35, and most preferably at least about 40 holes, per cm². The textile material can have at most 625, preferably at most 300, more preferably at most 100, and most preferably at most about 40 holes, per cm². The size of the holes may be at least 0.04 mm, preferably at least 0.05 mm, more preferably at least 0.06 mm, in average diameter. The size of the holes can be at most 2.0 mm, preferably at most 1.5 mm, more preferably at most 1.0 mm, and most preferably at most 0.08 mm, in average diameter.

In exemplary embodiments, the textile material of the transportation layer has a weight of at least 50 GSM (grams per square meter), preferably at least 80 GSM, more preferably at least 100 GSM, and most preferably at least about 120 GSM. The textile material can have a weight of at most 250 GSM, preferably at most 200 GSM, more preferably at least 150 GSM, and most preferably at most about 120 GSM.

In exemplary embodiments, the textile material of the transportation layer can have one or more of the following features. The textile material can have a water absorbency time of at most 5 seconds, preferably at most 3 seconds, more preferably at most 2 seconds, and most preferably at most 1 second, measured in accordance with AATCC test method 79-2014 (Option A). The textile material can have a water repellency rating of 0, measured in accordance with AATCC test method 22-2014. The textile material can exhibit a stain/oil repellency, measured in accordance with AATCC test method 118-2013, of at most rating 1, preferably rating o. The textile material can exhibit a fluid holding capacity of at least 5 times its weight, preferably at least 6 times its weight, more preferably at least 7 times its weight, and most preferably at least 8 times its weight, when tested according to ASTM D 7367 - 14. The textile material can exhibit a reduction value of *Escherichia coli ATCC 25922* and/or *Staphylococcus aureus ATCC 6538* and/or *Pseudomonas* aeruginosa *ATCC 15442* and/or *Salmonella enterica ATCC 10708* and/or *Candida albicans* and/or *Aspergillus Niger,* measured in accordance with AATCC test method 100-2012 and/or ASTM E2148 - 10, of at least 90% within 1 hour of contact time, preferably remaining at the same level or greater even after 8 hours, more preferably increasing to at least 99% (2 log) after 8 hours.

The textile material of the transportation layer can be produced using the process described above. In the process, the liquor applied in the main process cycle can contain organosilane terpolymers, preferably at least 20 gpl (grams per liter), more preferably at least 40 gpl, particularly at least 70 gpl, and most preferably at least about 100 gpl. The liquor applied in the main process cycle can contain an antimicrobial agent comprising carbendazim and isothiazoline, preferably at least 10 gpl (grams per liter), more preferably at least 25 gpl, particularly at least 40 gpl, and most preferably at least about 50 gpl. It is possible that the liquor applied in the main process cycle contains an acrylate-based cross linker, preferably at least 10 gpl (grams per liter), more preferably at least 25 gpl, particularly at least 40 gpl, and most preferably at least about 50 gpl. In the process, the curing dwell time of the main process cycle can be at least 20 sec, preferably at least 40 sec, more preferably at least 50 sec, and most preferably at least about 60 sec, per 60 GSM of fabric weight. The curing dwell time of the main process cycle can be at most 120 sec, preferably at most 100 sec, more preferably at most 80 sec, and most preferably at most about 60 sec, per 60 GSM of fabric weight. It is possible to use an exhaust process in the antimicrobial process cycle. The drying dwell time of the antimicrobial process cycle can be at least 20 sec, preferably at least 40 sec, more preferably at least 50 sec, and most preferably at least about 60 sec, per 60 GSM of fabric weight. The drying dwell time of the antimicrobial process cycle can be at most 120 sec, preferably at most 100 sec, more preferably at most 80

The textile material of the transportation layer can comprise at least 0.2%, preferably at least 0.4%, more preferably at least 0.6%, and most preferably at least about 0.8% of polyglucosamine, based on weight fabric (owf.). The textile material can comprise at least 0.05%, preferably at least 0.1%, more preferably at least 0.15%, and most preferably at least about 0.2% of silver cations, based on weight fabric (owf.).

The textile material of the transportation layer is preferably hydrophilic.

### Absorption layer(s) (layer 3)

In a preferred embodiment of the invention, the textile material is used in an absorption layer or constitutes an absorption layer. As used herein, absorption layer means the layer absorbing most of the body fluids. The absorption layer can be between the inner layer and the water repellant layer. If there is a transportation layer, the absorption layer can be between the transportation layer and the water repellant layer.

The textile material of the absorption layer may be a needle punch, spun bonded, melt blown non-woven and/or terry fleece fabric. The textile material may comprise at least 30%, preferably at least 45%, more preferably at least 60%, most preferably at least about 65% viscose. The textile material may comprise at most 100%, preferably at most 75%, more preferably at most 70%, most preferably at most about 65% viscose. The textile material may comprise at least 15%, preferably at least 25%, more preferably at least 30%, most preferably at least about 35% of a synthetic fiber, preferably polyester. The textile material may comprises at most 100%, preferably at most 65%, more preferably at most 40%, most preferably at most about 35% of a synthetic fiber. Preferably, the synthetic fiber is polyester. It is also possible to use polyamide or nylon as the synthetic fiber.

In exemplary embodiments, the textile material has a weight of at least 70 GSM (grams per square meter), preferably at least 100 GSM, more preferably at least 130 GSM, and most preferably at least about 150 GSM. The textile material can have a weight of at most 350 GSM, preferably at most 250 GSM, more preferably at most 170 GSM, and most preferably at most about 150 GSM.

In further exemplary embodiments, at least 95%, preferably at least 97%, more preferably at least 99%, of the textile material consist of a synthetic fiber, preferably polyester, and is a terry fleece, in particular a loop knitted toweling fabric.

The textile material can have a weight of at least 160 GSM, preferably at least 250 GSM, more preferably at least 290 GSM, and most preferably at least about 320 GSM. The textile material can have a weight of at most 450 GSM, preferably at most 400 GSM, more preferably at most 350 GSM, and most preferably at most about 320 GSM.

In order to support the absorbing effects, the textile material is preferably hydrophilic.

In further preferred embodiments, the textile material exhibits a stain release capability, measured in accordance with AATCC test method 130-2010, of at least rating 4, most preferably rating 5. These ratings may be achieved even after 25 washes in a laundry washing machine at 85±15 °C for 10-15 minutes, preferably using a non-antimicrobial, non-ionic and non-chlorine containing laundry detergent such as Tergitol 15-S-9 (from Dow Chemicals), preferably followed by a standard rinse cycle and preferably dried at 62-96 °C for 20-30 minutes. The textile material may exhibit a stain/oil repellency, measured in accordance with AATCC test method 118-2013, of at most rating 1, preferably rating o. In other embodiments, the textile material exhibits a fluid holding capacity of at least 6 times its weight, preferably at least 8 times its weight, more preferably at least 10 times its weight, and most preferably at least 12 times its weight, when tested according to ASTM D7367 - 14.

The textile material of the absorption layer may exhibit antimicrobial properties such that the textile material exhibits a reduction value of *Escherichia coli ATCC 25922* and/or *Staphylococcus aureus ATCC 6538* and/or *Pseudomonas aeruginosa ATCC 15442* and/or *Salmonella enterica ATCC 10708* and/or *Candida albicans* and/or *Aspergillus Niger,* measured in accordance with AATCC test method 100-2012 and/or ASTM E2149 - 10, of at least 99% (2 log), preferably at least 99.9% (3 log), within 1 hour of contact time, preferably remaining at the same level or greater even after 8 hours, more preferably increasing to at least 99.99% (4 log) after 8 hours. These reduction values may be achieved even after at least 25 laundry washes in a laundry washing machine at 85±15 °C for 10-15 minutes, preferably using non-antimicrobial, non-ionic and non-chlorine containing laundry detergent, e.g. Tergitol 15-S-9 (Dow Chemicals), preferably followed by a standard rinse cycle and preferably dried at 62-96 °C for 20-30 minutes.

The textile material of the absorption layer preferably comprises, as antimicrobial agents, chitosan, polyhexamethylene biguanide, silver cations, carbendazim and/or isothiazoline, in particular polyhexamethylene biguanide, silver cations, carbendazim and isothiazoline. The textile material can be produced using the process described above and using one or more of the following features. In the process, a liquor applied in the main process cycle can contain organosilane terpolymers, preferably at least 20 gpl (grams per liter), more preferably at least 40 gpl, particularly at least 70 gpl, and most preferably at least about 100 gpl. Preferably, in the process the curing dwell time of the main process cycle is at least 20 sec, preferably at least 40 sec, more preferably at least 50 sec, and most preferably at least about 60 sec, per 60 GSM of fabric weight, and the curing dwell time of the main process cycle is at most 120 sec, preferably at most 100 sec, more preferably at most 80 sec, and most preferably at most about 60 sec, per 60 GSM of fabric weight. In preferred embodiments, a padding process is used in the antimicrobial process cycle. In particular, the liquor applied in the antimicrobial process cycle contains polyglucosamine, preferably at least 4 gpl (grams per liter), more preferably at least 8 gpl, particularly at least 15 gpl, and most preferably at least about 20 gpl. The liquor applied in the antimicrobial process cycle may contain polyhexamethylene biguanide, preferably at least 4 gpl (grams per liter), more preferably at least 8 gpl, particularly at least 15 gpl, and most preferably at least about 20 gpl. The liquor applied in the antimicrobial process cycle may contain silver cations, preferably at least 0.2 gpl (grams per liter), more preferably at least 0.4 gpl, particularly at least 0.7 gpl, and most preferably at least about 1.0 gpl. The liquor applied in the antimicrobial process cycle may contain an antimicrobial agent comprising carbendazim and isothiazoline, preferably at least 10 gpl (grams per liter), more preferably at least 30 gpl, particularly at least 40 gpl, and most preferably at least about 50 gpl. The liquor applied in the antimicrobial process cycle contains an acrylate-based cross linker, preferably at least 10 gpl (grams per liter), more preferably at least 30 gpl, particularly at least 40 gpl, and most preferably at least about 50 gpl. In the process, the drying dwell time of the antimicrobial process cycle can be at least 20 sec, preferably at least 40 sec, more preferably at least 50 sec, and most preferably at least about 60 sec, per 75 GSM of fabric weight, and the drying dwell time of the antimicrobial process cycle can be at most 120 sec, preferably at most 100 sec, more preferably at most 80 sec, and most preferably at most about 60 sec, per 75 GSM of fabric weight.

### Water repellant layer (layer 4)

The water repellant layer is an outer layer preventing leakage of liquids or fluids. The textile material suitable as water repellant layer is not subject to any restrictions and can be any textile material known in the art for that purpose. In a preferred embodiment, a textile material produced using the above-described process is used in a water repellant layer or constitutes a water repellant layer.

In preferred embodiments, the textile material of the water repellant layer is woven and preferably a microfiber. In particular, at least 95%, preferably at least 97%, more preferably at least 99%, of the textile material may consist of polyester, nylon, polyamide, polypropylene, or any other synthetic textile substrates. Preferably, polyester is used. The textile material may have a weight of at least 50 GSM (grams per square meter), preferably at least 80 GSM, more preferably at least 110 GSM, and most preferably at least about 125 GSM. The textile material may have a weight of at most 250 GSM, preferably at most 200 GSM, more preferably at least 150 GSM, and most preferably at most about 125 GSM.

In a preferred embodiment, the textile material is waterproof.

The textile material of the water repellant layer can exhibit a stain/oil repellency, measured in accordance with AATCC test method 118-2013, of at least rating 4, preferably at least rating 5, more preferably at least rating 6. The textile material can exhibit a reduction value of *Escherichia coli ATCC 25922* and/or *Staphylococcus aureus ATCC 6538* and/or *Pseudomonas aeruginosa ATCC 15442* and/or *Salmonella enterica ATCC 10708* and/or *Candida albicans* and/or *Aspergillus Niger,* measured in accordance with AATCC test method 100-2012 and/or ASTM E2149 - 10, of at least 90% within 1 hour of contact time, preferably remaining at the same level or greater even after 8 hours, more preferably increasing to at least 99% (2 log) after 8 hours.

The textile material can have some antimicrobial properties. To achieve this, in the process, the liquor applied in the main process cycle can contain a quaternary ammonium organosilane compound, preferably at least 2 gpl (grams per liter), more preferably at least 4 gpl, particularly at least 6 gpl, and most preferably at least about 8 gpl. The liquor applied in the main process cycle can contain a fluorochemical, preferably at least 20 gpl (grams per liter), more preferably at least 40 gpl, particularly at least 60 gpl, and most preferably at least about 80 gpl. The process for producing the water repellant layer can comprise a curing dwell time of the main process cycle of at least 20 sec, preferably at least 40 sec, more preferably at least 50 sec, and most preferably at least about 60 sec, per 62.5 GSM of fabric weight. The process can comprise a curing dwell time of the main process cycle of at most 120 sec, preferably at most 100 sec, more preferably at most 80 sec, and most preferably at most about 60 sec, per 62.5 GSM of fabric weight.

In a specific embodiment, the textile material is obtainable by a process comprising a water repellency process cycle comprising the steps of:
- treating a textile material using an application process such as a knife or screen coating process, a spraying process, an exhaust process, or preferably a padding process, wherein a liquor is applied to the textile which comprises one or more chemical agents which increase water repellency of the textile material, and
- subjecting the treated textile material to a heat treatment.

It is possible that the water repellency process cycle is performed before the main process cycle.

In preferred embodiments, the liquor applied in the water repellency process cycle contains an agent comprising C6- or C8-fluorocarbon and/or polyurethane (PU), preferably at least 20 gpl (grams per liter), more preferably at least 40 gpl, particularly at least 60 gpl, and most preferably at least about 80 gpl. The heat treatment of the water repellency process cycle may comprise drying conducted at least partially at a temperature of at least 70°C, preferably at least 100°C, more preferably at least 110°C, most preferably at least about 120°C. The heat treatment may comprise drying conducted at least partially at a temperature of at most 160°C, preferably of at most 140°C, more preferably of at most 130°C, and most preferably of at most about 120°C. The drying dwell time of the water repellency process cycle may be at least 20 sec, preferably at least 40 sec, more preferably at least 50 sec, and most preferably at least about 60 sec, per 62.5 GSM of fabric weight. The drying dwell time of the water repellency process cycle may be at most 120 sec, preferably at most 100 sec, more preferably at most 80 sec, and most preferably at most about 60 sec, per 62.5 GSM of fabric weight.

The textile material of the inner layer, the transportation layer, the absorption layer, and/or the water repellant layer has the above-described properties, such as stain release capability, antimicrobial efficiency, water holding capacity, water absorption time, stain/oil repellency, or water repellency in a wash-durable manner. In particular, said properties are achieved after many washes, in particular even after at least 25 laundry washes in a laundry washing machine at 85±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

### Hygiene product

A preferred embodiment of the invention is a hygiene product. The hygiene product comprises at least one layer, preferably at least two layers, more preferably three layers or at least three layers. The one or more layers can comprise, consist, or be formed by a textile material. The textile material can convey stain release capability, antimicrobial efficiency, water holding capacity, water absorption time, stain/oil repellency, or water repellency, in particular stain release, absorbing, and/or antimicrobial properties. In exemplary embodiments, the hygiene product comprises one layer, preferably formed by a textile material, and at least a second layer formed by a textile material conveying stain release, absorbing and/or antimicrobial properties. In further embodiments, the hygiene product comprises a further layer of a textile material. The layer, the second layer and/or the further layer of the textile material may comprise or consist of a synthetic fabric or a natural fabric or a blend of both, with or without the addition of a stretch yarn, in particular lycra or spandex. In exemplary embodiments, the textile material comprises polyester, preferably consists of polyester. In other exemplary embodiments, the textile material comprises a blend of polyester and cotton, preferably consists of a blend of polyester and cotton, e.g. about 65% polyester and about 35% cotton.

In exemplary embodiments of the hygiene product, at least one of the surfaces of the further layer is finished with a water-and-oil repellant, optionally including polyurethane (PU). In other exemplary embodiments, the further layer is formed by a textile material which is a blend of polyester and cotton, e.g. about 65% polyester and about 35% cotton.

In further exemplary embodiments, the hygiene product comprises three layers, wherein two layers are formed by a textile material conveying stain release, absorbing and/or antimicrobial properties, and the third layer is impermeable to liquids, e.g. the third layer is formed by a textile finished with a water- and-oil repellant. The layers of a hygiene product may be connected by ultra-sound welding, stitching, gluing or by gluing using fusible interlinings.

The hygiene product can be a sanitary napkin. In another embodiment, the hygiene product can be a sanitary lungot or sanitary langot. Further, exemplary hygiene products are panty or underwear liners, tampons, nappies, diapers, diaper liners, adult diapers, panties or underwear, bras, or nursing pads. Other exemplary embodiments are a towel, kitchen towel, napkin, handkerchief, face mask, floor cleaning mop, wipe, or textile used in wound care. As an exemplary embodiment, the hygiene product may be used as a hand sanitizer.

In a preferred embodiment, a hygiene product comprises a textile material as described above. The textile material can form a layer. The hygiene product can comprise at least a second layer formed by a textile material as described above. The hygiene product can comprise a further layer of a textile material. The giene product of claim 171, wherein the further layer comprises or consists of a synthetic fabric or a natural fabric or a blend of both, with or without the addition of a stretch yarn, in particular lycra or spandex. At least one of the surfaces of the further layer may be finished with a water- and oil-repellant, optionally including polyurethane (PU), preferably enhanced with perfluoroalkylethylacrylate copolymer with C6 or and C8 carbon chain. The hygiene product may be one selected from the group consisting of sanitary napkins, sanitary lungots or lungots, panty or underwear liners, tampons, nappies, diapers, diaper liners, adult diapers, panties or underwear, bras, bed pads, or nursing pads. Further, the hygiene product may be a towel, napkin, handkerchief, face mask, floor cleaning mop, wipe, or textile used in wound care, comprising or consisting of a textile material as described above.

The hygiene product of the invention provides the advantage that due to its stain release properties it can be washed easily at mild conditions, e.g. with water, optionally using some mild detergent, by hand or in a washing machine at low temperatures, e.g. 30°C or 40°C.

### Sanitary napkin

A preferred embodiment of the invention is a sanitary napkin. The sanitary napkins comprises one or more layers comprising or consisting of a textile material conveying stain release capability, antimicrobial efficiency, water holding capacity, water absorption time, stain/oil repellency, or water repellency, in particular stain release, absorbing and/or antimicrobial properties, and a further layer having water repellant properties. At least one of the surfaces of the further layer can be finished with a water-and-oil repellant, optionally including polyurethane (PU). The textile material can convey stain release and absorbing properties; stain release and antimicrobial properties; absorbing and antimicrobial properties; or stain release, absorbing and antimicrobial properties.

The sanitary napkin can comprise one or more layers comprising polyester textile, in particular an inner layer wherein the polyester textile is finished to exhibit stain release, antimicrobial and/or absorbing properties. In further exemplary embodiments, the sanitary napkin comprises one or more layers comprising terry fleece, in particular polyester terry fleece. The sanitary napkin may comprise a middle layer comprising polyester terry fleece, in particular a loop knitted toweling fabric brushed to give a fleece effect, exhibiting stain release, antimicrobial and absorbing properties
In exemplary embodiments, the sanitary napkin comprises three layers, namely an inner layer, a middle layer and an outer layer, wherein one surface of the middle layer adjoins one surface of the inner layer.

The sanitary napkin may comprise a further layer comprising or consisting of a textile material at least one surface of which is coated with a water-and-oil repellant, optionally including polyurethane (PU).

In exemplary embodiments of the sanitary napkin, one or more layers comprise a blend of polyester and cotton. In other exemplary embodiments of the sanitary napkin, one or more layers comprises polyester, preferably 50 to 100 % by weight, in particular 100 % by weight.

The sanitary napkin may comprise an outer layer and middle layer, wherein in particular one surface of the middle layer adjoins one surface of the outer layer. The middle layer can comprise two or more, in particular two layers, depending on the desired absorption capacity of the sanitary napkin. Two middle layers can form a transport layer and an absorption layer as described above. The absorption layer may comprise or consist of one, two or more layers. In preferred embodiments, the surface of the outer layer adjoining the middle layer is coated with a water-and-oil repellant, and the other surface of the outer layer is coated with a water-and-oil repellant including polyurethane (PU), optionally treated with a quaternary ammonium organosilane compound.

In a preferred embodiment, the sanitary napkin comprises one or more dispersion layers comprising or consisting of textile material, and a water repellent layer having a water repellency rating of at least 70, preferably at least 80, more preferably at least 90, measured in accordance with AATCC test method 22-2014. The one or more dispersion layers comprise an inner layer comprising a textile material as described above. The inner layer is the layer which lies against the skin of the user when the sanitary napkin is worn by the user. The one or more dispersion layers can comprise one or more main absorption layers, each main absorption layer comprising a textile material as described above. The one or more main absorption layers are located between the inner layer and the water repellent layer. The sanitary napkin can comprise two main absorption layers. The one or more dispersion layers can comprise a transportation layer comprising a textile material as described above. The transportation layer may be located between the inner layer and the one or more main absorption layers. The water repellent layer can be a textile material as described above. The water repellent layer can be the water repellant layer of the sanitary napkin.

The one or more dispersion layers of the sanitary napkin in conjunction can have fluid dispersion properties such that if 1 ml of water is dropped onto the dispersion layers, the water is dispersed within 1 sec in a dispersion area having an average diameter of at least 3 cm, preferably at least 4 cm, more preferably at least 4.5 cm, and most preferably at least 5 cm.

The sanitary napkin can have the shape of a pad. The pad can have a width in the range of from 3 cm to 20 cm. The pad can have a length in the range of 50 cm to 100 cm. The pad can have a width in the range of from 3 cm to 10 cm. The pad can have a length in the range of 10 cm to 50 cm. The pad can comprise fixing means to attach the pad to a textile. The sanitary napkin of any one of claims 176 to 193, having the shape of a strip, wherein the water repellent layer has a width in the range of from 5 cm to 20 cm and a length in the range of from 10 cm to 100 cm, and the dispersion layers are attached to said water repellent layer, and each of the dispersion layers has a width in the range of from 5 cm to 20 cm and a length in the range of from 10 cm to 100 cm. In the sanitary napkin, the water repellent layer and the dispersion layers substantially can have the same width. Preferably, the water repellent layer has a greater length than the dispersion layers. The dispersion layers can be attached to said water repellent layer by stitching, gluing, or preferably by ultrasonic welding.

The sanitary napkin can comprise at least one ribbon at each of the opposing edges of at least one longitudinal end of the water repellent layer. The ribbons can be attached by stitching, glueing, or preferably ultrasonic welding. The sanitary napkin can be a lungot or langote.

Other embodiments of the sanitary napkin are the same embodiments as disclosed herein for the hygiene products.

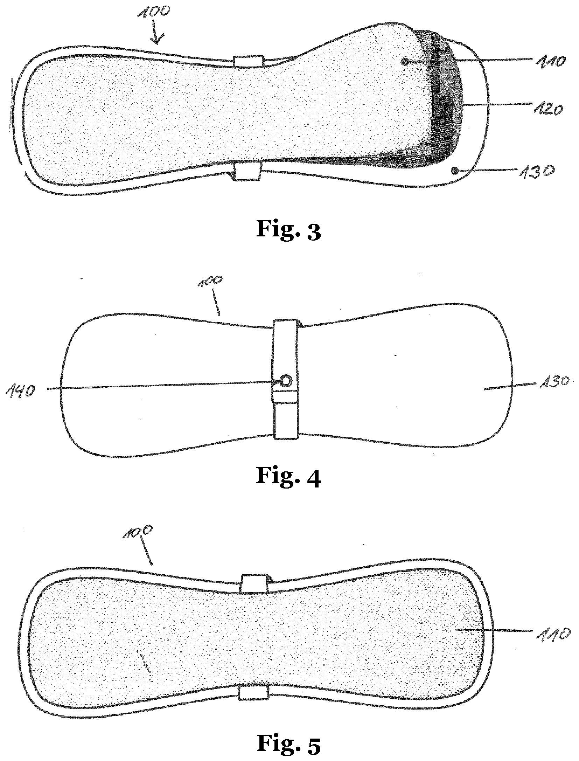

The sanitary napkin can have the shape of a pad. An exemplary embodiment is shown in Figures 3, 4 and 5. Fig. 3 shows a pad (100) comprising three layers (110), (120), (130). Layer (110) is the top layer or inner layer (i.e. the layer next to the skin). The layer (110) transports body discharges, e.g. blood, to a layer (120). Layer (110) is formed from a textile material conveying in particular stain release, and antimicrobial properties. Layer (120) is the middle layer, which retains and absorbs the body discharges. Layer (120) shown as one layer can comprise two or more, in particular two layers, depending on the desired absorption capacity of the sanitary napkin. If there are two middle layers, these can form a transport layer and an absorption layer as described above. The absorption layer may comprise or consist of one, two or more layers. Layer (130) is the bottom layer or outer layer, which prevents staining and a leakage of the retained body discharges. Preferably, layer (130) is formed by a textile material finished with water- and oil-repellant, optionally further finished with PU coating. Fig. 4 shows the pad (100) of Fig. 3 from the back side with the bottom layer (130). According to Fig. 4 the pad (100) can comprise a snap button (140). In other embodiments, the button (140) can be a metal or plastic button, or can be replaced with a hook-and-loop-fastener or Velcro. Fig. 5 shows the pad (100) of Fig. 3 from the front with the top layer (110). It is further possible that the pad comprises an inbuilt absorption pad as a further pad.

In exemplary embodiments, the pad has a width in the range of from 3 cm to 20. In other embodiments, the pad has a length in the range of 10 cm to 100 cm or a length in the range of 50 cm to 100 cm. In further embodiments, the pad has a width in the range of from 3 cm to 10 cm.

The pad may comprise fixing means, e.g. self-adhesive strip or hook-and-loop fastener, to attach the pad to a textile or undergarment. A further fixing means can be a snap button as shown in Fig. 4.

In other embodiments, the sanitary napkin has the shape of a strip. Such an embodiment is also called lungote, lungot, langote, or langot. In preferred embodiments, the strip has three layers, an inner layer, a middle layer and an outer layer. In exemplary embodiments, the outer layer has a width in the range of from 5 cm to 20 cm and a length in the range of from 10 cm to 100 cm, the middle layer and inner layer are attached to said outer layer, and each of the middle layer and inner layer has a width in the range of from 5 cm to 20 cm and a length in the range of from 10 cm to 100 cm. In other embodiments, the outer layer, middle layer and inner layer substantially have the same width. In further embodiments, the outer layer has a larger length than the middle layer and inner layer. Generally, the strip has such a size that it fits to a wearer.

The layers of the sanitary napkin may be connected by stitching, ultrasound-welding, gluing or by using fusible interlinings. In preferred embodiments, the middle layer and the inner layer are connected with the outer layer by stitching gluing, or by using fusible interlinings or ultrasound welding.

For fixing the sanitary napkin to the body of a wearer, at least one ribbon or string is provided at each of the opposing edges of at least one longitudinal end of the outer layer.

### Lungote or Langote

Preferred embodiments of the invention are sanitary napkins in form a strip, also called lungot, lungote, langot or langote. The latter four expressions are used interchangeable. The lungote can comprise an outer layer, a middle layer and an inner layer, wherein one surface of the middle layer adjoins one surface of the inner layer, and the other surface of the middle layer adjoins one surface of the outer layer. Depending on the desired absorption capacity, the middle layer can comprise two or more layers, preferably two layers.

Preferably, the outer layer has a width in the range of from 5 cm to 20 cm, e.g. 10 cm or 15 cm, and a length in the range of from 10 cm to 100 cm, e.g. 30 cm or 50 cm. In preferred embodiments, the middle layer and inner layer are attached to said outer layer, and each of the middle layer and inner layer has a width in the range of from 5 cm to 20 cm, e.g. 10 cm or 15 cm, and a length in the range of from 10 cm to 100 cm, e.g. 30 cm or 50 cm. The inner layer and the middle layer each can comprise or consist of a textile material conveying stain release, absorbing and/or antimicrobial properties. The outer layer can comprise or consist of a textile material being coated with a water-repellant, optionally including polyurethane (PU). The middle layer and the inner layer are attached to said outer layer, preferably by stitching.

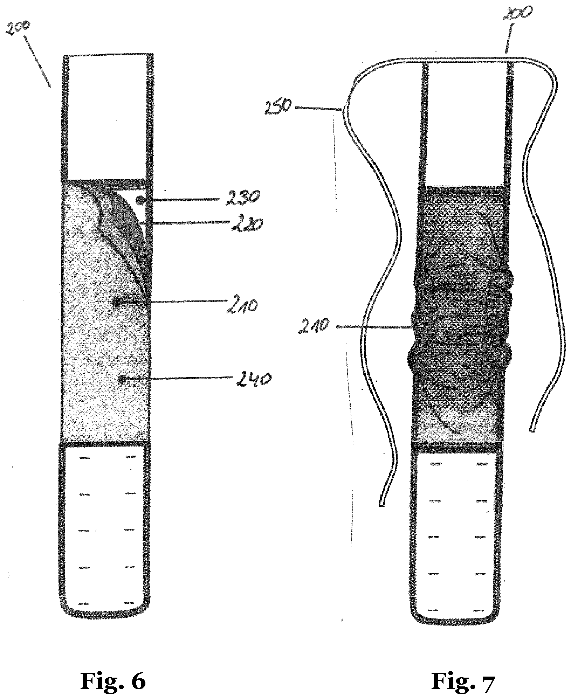

Exemplary embodiments of a lungote are shown in Figures 6, 7 and 8. Fig. 6 shows a lungote (200) having three layers (210), (220) and (230). The layer (210) is the top layer or inner layer. Layer (210) corresponds to layer (110) of Fig. 3. The layer (220) is the middle layer and corresponds to layer (120) of Fig. 3. The layer (230) is the bottom layer or outer layer and corresponds to layer (130) of Fig. 3. According to Fig. 6 the lungot further comprises an inbuilt pad (240).

Fig. 7 shows a lungot (200) with top layer (210) and a string or ribbon (250) for attaching or fixing the lungot to the body of the wearer.

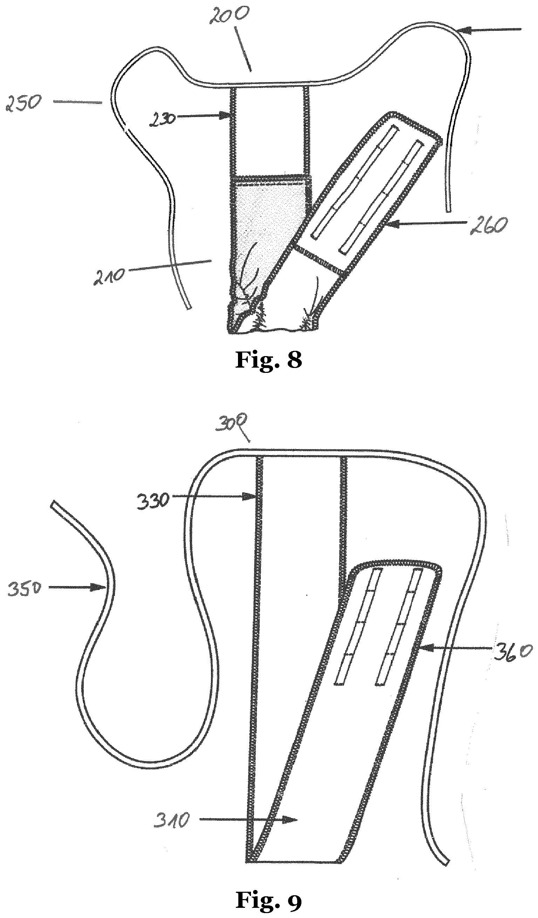

Fig. 8 shows a lungot (200) with top layer (210) and string (250). Further shown are the bottom layer (230) and by reference numeral (260) a front with adjustable loops. The lungot has loops so that the size is adjustable. The back side of the lungot has two strings that come around the waist and, depending on the size, can be adjusted to the wearer by using the loops in the front.

Another embodiment is a strip, lungote or langote comprising an outer layer having a width in the range of from 5 cm to 20 cm, e.g. 10 cm or 15 cm, and a length in the range of from 10 cm to 100 cm, e.g. 30 cm or 50 cm, wherein said outer layer comprises or consists of a textile material being coated with a water-repellant, optionally including polyurethane (PU). The lungot or langote can comprise at least one ribbon or string at each of the opposing edges of at least one longitudinal end of the outer layer.

An exemplary embodiment is shown in Fig. 9. In Fig. 9, a lungot (300) comprises one layer with reference numeral (330) designating the back side and reference numeral (310) the front side. Like the lungot (200) shown in figures 6, 7 and 8, the lungot in Fig. 9 has a front with adjustable loops designated with reference numeral (360).

A pad as described above can be fixed or attached to the lungot. The pad can the same pad as described above in the context of the sanitary napkin or shown in figures 3, 4 and 5.

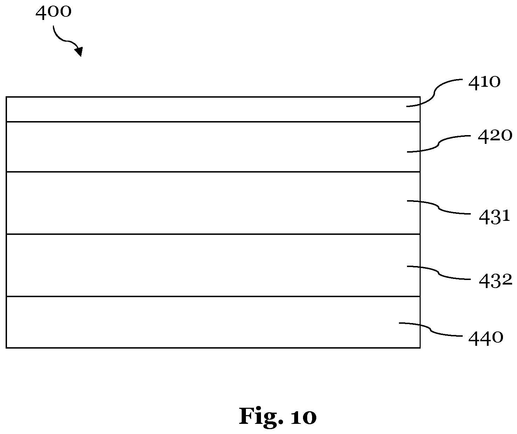

Fig. 10 shows one embodiment of the invention with a layer construction 400, with inner layer 410, transportation layer 420, two absorption layers 431 and 432, and water repellant layer 440. In other embodiments, only one absorption layer may be provided. In further embodiments, more than two layers may be provided. It will be appreciated that transportation layer 420 may be left away.

### Other products

The textile materials obtained by using the process described herein can be used to produce other products, such as towel, kitchen towel, napkin, handkerchief, face mask, floor cleaning mop, wipe, textiles used in wound care.

The invention will be further described by the following example, which illustrates preferred embodiments without limiting the scope of the invention.

### EXAMPLES

### Example 1: Sanitary napkin

A sanitary napkin comprising four layers of textile material was produced. The sanitary napkin was produced as described hereinafter and as summarized in Table 1 hereinafter.

In Table 1, the abbreviations used have the following meanings:
- organosilane: quaternary ammonium organosilane compound
- chitosan: polyglucosamine
- PHMB: polyhexamethylene biguanide
- Silvadur: silver cations
- Afrotin: carbendazim and isothiazoline
- Ukadan cross linker: acrylate- based crosslinker
- Hydrosil: organosilane terpolymer
- Globe PUWP: water repellant with polyurethane
- AG 7600 or equivalent: water repellant

These abbreviations also apply to the following description.

The inner layer, layer 1, (i.e. the layer closest to the body) was produced from a textile material being 100% polyester, fine mesh, treated with quaternary ammonium organosilane compound. Particularly, the textile material being 100% polyester was of fine mesh appearance, provided as knitted monofilament, with a specific weight of 60 gram per square meter. Then, the textile material of layer 1 was processed with an exhaust process. The exhaust liquor comprises organosilane in an amount of 0.8% on weight of fabric, as well as silvadur in an amount of 0.2% on weight of fabric. During the exhaust process, the liquor had a temperature of 80°C, and the exhaust process time was 60 minutes. Afterwards the treated material was dried at 120 °C for 30 seconds. Following this first process cycle a padding process was applied, with the padding liquor comprising chitosan in an amount of 10 grams per liter, afrotin in an amount of 50 grams per liter, and ukadan cross linker in an amount of 50 grams per liter. The liquor pickup rate during the padding process was 100%. Afterwards a curing process was performed at 180°C for 60 seconds. Thus, the process steps of exhaustion, drying, padding and curing were performed in sequence for finishing layer 1.

The resulting finished layer 1 features a mildy hydrophobic function. It has a water repellency rating of at least 70, preferably at least 80, more preferably at least 90, measured in accordance with AATCC test method 22-2014; a water absorbency time of at least 2 minutes, preferably at least 3 minutes, measured in accordance with AATCC test method 79-2014 (Option A), and a water holding capacity of less than 2 times its weight as tested according to ASTM D7367-14. It exhibits a stain release capability of a rating larger than 5 as measured in accordance with AATCC test method 130-2010. Also, resulting layer 1 exhibits a stain/oil repellency capability of a rating larger than 2 as measured in accordance with AATCC test method 118-2013. It, moreover, exhibits a microbial reduction value of *Escherichia coli* ATCC 25922, *Staphylococcus aureus* ATCC 6538, *Pseudomonas aeruginosa ATCC 15442, Salmonella enterica* ATCC 10708, *Candida albicans,* and *Aspergillus Niger* as measured in accordance with AATCC test method 100-2012 and ASTM test method E2149 -10 of more than 99.9% (3 log) within 1 hour of contact time.

In a preferred embodiment, layer 1 may form inner layer 410 of Fig. 10.

Layer 2 was produced from a textile material being 100% polyester, provided as a warp knit multi filament with impression fabric appearance, with a specific weight of 120 grams per square meter. Again, the textile material was subjected to an exhaust process. The exhaust liquor now comprises chitosan in an amount of 0.8% on weight of fabric and silvadur in an amount of 0.2% on weight of fabric. During the exhaust process, the liquor had a temperature of 80°C, and the exhaust process time was 60 minutes. Afterwards the treated material was dried at 120 °C for 120 seconds. Following this first process cycle a padding process was applied, with the padding liquor comprising afrotin in an amount of 50 grams per liter, ukadan cross linker in an amount of 50 grams per liter and hydrosol in an amount of 100 grams per liter. The liquor pickup rate during the padding process was 100%. Afterwards a curing process was performed at 180°C for 120 seconds. Thus, the process steps of exhaustion, drying, padding and curing were performed in sequence for finishing layer 2.

The resulting finished layer 2 features a hydrophilic function. It has a water absorbency time of at most 5 seconds, preferably at most 3 seconds, more preferably at most 2 seconds, and most preferably at most 1 second, measured in accordance with AATCC test method 79-2014 (Option A), a water repellency rating of o, measured in accordance with AATCC test method 22-2014, and a water holding capacity of more than 8 times its weight as tested according to ASTM D7367-14. It exhibits a stain release capability of a rating larger than 5 as measured in accordance with AATCC test method 130-2010. The stain/oil repellency capability of resulting layer 2 has a rating of zero as measured in accordance with AATCC test method 118-2013. 1 ml of water dropped onto the layer disperses within 1 sec in a dispersion area having a diameter more than 5 cm. It, moreover, exhibits a microbial reduction value of *Escherichia coli* ATCC 25922, *Staphylococcus aureus* ATCC 6538, *Pseudomonas aeruginosa ATCC 15442, Salmonella enterica* ATCC 10708, *Candida albicans,* and *Aspergillus Niger* as measured in accordance with AATCC test method 100-2012 and ASTM test method E2149-10 of more than 99.9% (3 log) within 1 hour of contact time.

In a preferred embodiment, layer 2 may form transportation layer 420 of Fig. 10.

Layer 3 was produced from a textile material being a blend of 65% viscose and 35% polyester, provided as a needle punch non-woven material, with a specific weight of 150 grams per square meter. Alternatively also a 100% poly fleece material can be chosen. The textile material was first treated with a padding process. The padding liquor comprises chitosan in an amount of 20 grams per liter, PHMT in an amount of 20 grams per liter, silvadur in an amount of 1 gram per liter, afrotin in amount of 50 grams per liter, and ukadan cross linker in an amount of 50 grams per liter. The liquor pickup rate during the padding process was 100%. Afterwards the treated material was dried at 120 °C for 120 seconds. Following this first process cycle a second padding process was applied, with the padding liquor comprising hydrosol in an amount of 100 grams per liter. The liquor pickup rate during the second padding process was 100%. Afterwards a curing process was performed at 180°C for 120 seconds. Thus, the process steps of padding, drying, padding and curing were performed in sequence for finishing layer 3.

The resulting finished layer 3 features a hydrophilic function. It has a water absorbency time of at most 5 seconds, preferably at most 3 seconds, more preferably at most 2 seconds, and most preferably at most 1 second, measured in accordance with AATCC test method 79-2014 (Option A), a water repellency rating of o, measured in accordance with AATCC test method 22-2014, and a water holding capacity of more than 8-12 times its weight as tested according to ASTM D7367-14. It exhibits a stain release capability of a rating larger than 5 as measured in accordance with AATCC test method 130-2010. The stain/oil repellency capability of resulting layer 3 has a rating of zero as measured in accordance with AATCC test method 118-2013. 1 ml of water dropped onto the layer disperses within 1 sec in a dispersion area having a diameter more than 5 cm. It, moreover, exhibits a microbial reduction value of *Escherichia coli* ATCC 25922, *Staphylococcus aureus* ATCC 6538, *Pseudomonas aeruginosa ATCC 15442, Salmonella enterica* ATCC 10708, *Candida albicans,* and *Aspergillus Niger* as measured in accordance with AATCC test method 100-2012 and ASTM test method E2149-10 of between 99% (log 2) and 99.9% (3 log) within 1 hour of contact time.

In a preferred embodiment, layer 3 may form any one of absorption layers 431 and 432 of Fig. 10.

Layer 4 was produced from a textile being 100% polyester. The textile material was woven and had a microfiber woven appearance. The specific weight was 125 grams per square meter. The textile material was first treated with a padding process. The padding liquor comprises globe PUWP, i.e. a water repellant with polyurethane in an amount of 500 grams per liter. The liquor pickup rate during the padding process was 100%. Afterwards the treated material was dried at 120 °C for 120 seconds. Following this first process cycle a second padding process was applied, with the padding liquor comprising organosilane in an amount of 8 grams per liter, and the water repellant AG 7600 in an amount of 80 grams per liter. The liquor pickup rate during the second padding process was 100%. Afterwards a curing process was performed at 180°C for 120 seconds. Thus, the process steps of padding, drying, padding and curing were performed in sequence for finishing layer 4.

The resulting finished layer 4 features water proof functionality. It exhibits a stain release capability of a rating larger than 5 as measured in accordance with AATCC test method 130-2010. The stain/oil repellency capability of resulting layer 4 has a rating of larger than 5 as measured in accordance with AATCC test method 118-2013. The fluid dispersion test is not applicable to resulting layer 4. Also, it exhibits a water holding capacity of zero times its weight as tested according to ASTM D7367-14. The antimicrobial properties of resulting layer 4 are a microbial reduction value of *Escherichia coli* ATCC 25922, *Staphylococcus aureus* ATCC 6538, *Pseudomonas aeruginosa ATCC 15442,* and *Salmonella enterica* ATCC 10708 of 99% (log 2) within 8 hours of contact time as measured in accordance with AATCC test method 100-2012 and ASTM test method E2149-10 and a microbial reduction value of *Candida albicans* and *Aspergillus Niger* of less than ∼ 30% (log 0.5) within 8 hour of contact time as measured in accordance with AATCC test method 100-2012 and ASTM test method E2149-10.

In a preferred embodiment, layer 4 may form water repellant layer 440 of Fig. 10.

**Table 1**

| **Specification** | **Layer 1** | **Layer 2** | **Layer 3** | **Layer 4** |
|---|---|---|---|---|
| Visual Discription | Fine Mesh | Impression Fabric | 60 GSM | Microfibre Woven |
| Construction | Knitted Monofilament | Warp Knit Multi Filament | Needle Punch non Woven | Woven |
| Composition | 100% Poly | 100% Poly | 65% Viscose/35% Poly | 100% Poly |
| Weight | 60 GSM | 120 GSM | 150 GSM (x2 option) | 125 GSM |
| Basic Function | Mildy Hydrophobic | Hydrophillic | Hydrophillic | Water Proof |
| Stain Release AATCC 130 | >5 | >5 | >5 | >5 |
| Stain/OIL Repellant AATCC 118 | >2 | 0 | 0 | >5 |
| Fluid Dispersion | 1ml to be absrobed instantaneously (<1 Sec), and spread >5 cm in 1 Sec | | | Not applicable |
| Fluid retention to weight | < 2 times | >8 times | >8 times | 0 |
| **Antimicrobial Performance** | | | | |
| When tested as per AATCC 100 | | | | |
| E.Coli AATCC 25922 | >3 Logs in 1 Hour | >1 but < 2 log in 1 Hour | >3 Logs in 1 Hour | = 2 Logs in 8 Hours |
| S.Aureus AATCC 6538 | >3 Logs in 1 Hour | >1 but < 2 log in 1 Hour | >3 Logs in 1 Hour | = 2 Logs in 8 Hours |
| P.Aureginosa AATCC 15442 | >3 Logs in 1 Hour | >1 but < 2 log in 1 Hour | >3 Logs in 1 Hour | = 2 Logs in 8 Hours |
| S.Enterica AATCC 10708 | >3 Logs in 1 Hour | >1 but < 2 log in 1 Hour | >3 Logs in 1 Hour | = 2 Logs in 8 Hours |
| Candida Albicans | >3 Logs in 1 Hour | >1 but < 2 log in 1 Hour | >3 Logs in 1 Hour | <0.5 log in 8 Hours |
| A.Niger | >3 Logs in 1 Hour | >1 but < 2 log in 1 Hour | >3 Logs in 1 Hour | <0.5 log in 8 Hours |
| | | | | |
| **Recipie** | | | | |
| **By exhaust 80 Deg / 60 Minutes** | | | Not Applicable | Not Applicable |
| Organosilane | 0.8% OWF | 0 | | |
| Chitosan | Nil | 0.8% OWF | | |
| PHMB | Nil | 0 | | |
| Silvadur | 0.2% OWF | 0.2% OWF | | |
| Afrotin | Nil | 0 | | |
| **Drying at 120 Deg C** | 30 Sees | 2 Minutes | | |
| | | | | |
| **By Padding 100% Pickup** | **Not applicable** | **Not applicable** | | |
| Organosilane | | | 0 | |
| Chitosan | | | 20 GPL | |
| PHMB | | | 20 GPL | |
| Silvadur | | | 1 GPL | |
| Afrotin | | | 50 GPL | |
| Ukadan Cross Linker | | | 50 GPL | |
| Hydrosil | | | | |
| Globe PUWP | | | | 500 GPL |
| AG 7600 or Equivalent | | | | |
| **Drying at 120 Deg C** | | | 2 Minutes | 2 Minutes |
| | | | | |
| **By Padding 100% Pickup** | | | | |
| Organosilane | | | | 8 GPL |
| Chitosan | 10 GPL | | | |
| PHMB | | | | |
| Silvadur | | | | |
| Afrotin | 50 GPL | 50 GPL | | |
| Ukadan Cross Linker | 50 GPL | 50 GPL | | |
| Hydrosil | | 100 GPL | 100 GPL | |
| Globe PUWP | | | | |
| AG 7600 or Equivalent | | | | 80 GPL |
| **Curing at 180 C** | 1 Minute | 2 Minutes | 2 Minutes | 2 Minutes |

Example 2: Disinfecting textile for application in diapers for incontinence (not according to the invention)

First, select a textile material comprising 100% cotton or 100% viscose or a blend of cotton and polyester or a blend of viscose and polyester.

Spray technique is used for application.

Chemicals used: 0.2 g/l silver chloride in aluminosilicate carrier base, 5 g/l polyhexamethylene biguanide, 10 g/l propiconozole, and 0.03% citric acid for adjusting pH-value between 5 and 6.

All the chemicals are dissolved in water and fed into the drum of a spray gun. The textile material is then sprayed at room temperature. Following this, the material is dried with a hot air gun at 180 °C for 2 minutes.

Example 3: Disinfecting textile for use as a hand sanitizer

First, select a textile material comprising of 100% cotton or 100% viscose or a blend of cotton and polyester or a blend of viscose and polyester.

Then prepare chemicals in a liquor, and feed into bath of exhaust machine. Fabric is dried at 120 °C using a stenter frame.

Subsequent the fabric is put through a padding mangle at room temperature with chemicals and then cured at 180 °C. The fabric is then cut into required pieces and used as a napkin or handkerchief with hand sanitizing properties.

Chemicals used in exhaust: 0.2% OWF silver chloride in aluminosilicate carrier base, 1 % OWF of polyhexamethylene biguanide, 0.8% OWF of polyglucosamine and 0.03% citric acid for adjusting pH-value between 5 and 6, along with water (OWF means on weight of fabric).

Chemicals used in padding : 50 GPL of organosilane terpolymers, 50 GPL of carbendazime complex and 50 GPL of a cross linker based on acrylates (GPL means grams per liter).

### Example 4. Disinfecting and stain release bed pads

A bed pad can be produced by proceeding in the same manner as described above in Example 1 for the sanitary pad.

## Claims

1. A process of finishing a textile material comprising an antimicrobial process cycle and a main process cycle, the antimicrobial process cycle being performed before the main process cycle,
wherein the antimicrobial process cycle comprises the steps of:
- treating the textile material using an exhaust process, wherein the liquor comprises one or more chemical agents which increase the antimicrobial efficacy of the textile material,
- subjecting the treated textile material to a heat treatment,
and wherein the main process cycle comprises the steps of:
- treating the textile material using an application process such as a knife or screen coating process, a spraying process, an exhaust process, or preferably a padding process, wherein a liquor is applied to the textile which comprises one or more chemical agents which increase the stain release capability and the water holding capacity of the textile material, the chemical agents which increase the stain release capability and the water holding capacity of the textile material being based on at least one selected from the group consisting of carboxylic acid esters, organosilane terpolymers, and starch based emulsion, and
- subjecting the treated textile material to a heat treatment.

2. The process of claim 1, wherein one or more chemical agents which increase the antimicrobial efficacy of the textile material are selected from the group consisting of quaternary ammonium organosilane compounds, silver cations, polyglucosamine, propiconazole, and polyhexamethylene biguanide, in particular of silver cations, propiconazole and/or carbendazim and isothiazoline.

3. The process of any one of claims 1 to 2, wherein the heat treatment of the antimicrobial process cycle comprises drying conducted at least partially at a temperature of at least 70°C, preferably at least 100°C, more preferably at least 110°C, most preferably at least about 120°C, and at a temperature of at most 160°C, preferably of at most 140°C, more preferably of at most 130°C, and most preferably of at most about 120°C.

4. The process of any one of claims 1 to 3, wherein the heat treatment of the main process cycle comprises curing of the textile material, wherein the curing is conducted at least partially at a temperature of at least 160°C, preferably at least 170°C, more preferably at least 175°C, and most preferably at least about 180°C, and at a temperature of at most 200°C, preferably at most 190°C, more preferably at most 185°C, and most preferably at most 180°C.

5. A textile material having a stain release capability, measured in accordance with AATCC test method 130-2010, of at least rating 3, preferably at least rating 4, and most preferably rating 5,
wherein the textile material exhibits a reduction value of *Escherichia coli* ATCC 25922 and/or *Staphylococcus aureus* ATCC 6538 and/or *Pseudomonas aeruginosa* ATCC 15442 and/or *Salmonella enterica* ATCC 10708 and/or *Candida albicans* and/or *Aspergillus Niger,* measured in accordance with AATCC test method 100-2012 and/or ASTM E2149 - 10, of at least 90% (1 log), preferably at least 99% (2 log), more preferably at least 99.9% (3 log), within 1 hour of contact time, preferably remaining at the same level or greater even after 8 hours, more preferably increasing to at least 99.99% (4 log) within 8 hours, and
wherein the textile material has a water holding capacity of at least 3 times its weight, preferably at least 5 times its weight, more preferably at least 7 times its weight, in particular at least 8 times its weight, most preferably at least 12 times its weight, preferably when tested according to ASTM D7367 - 14, and/or wherein the textile material has a water absorbency time of at most 5 seconds, preferably at most 3 seconds, more preferably at most 2 seconds, and most preferably at most 1 second, measured in accordance with AATCC test method 79-2014 (Option A).

6. The textile material of claim 5, wherein one or more chemical agents are fixed to the textile material which increase the stain release capability of the textile material, the one or more chemical agents being based on at least one selected from the group consisting of carboxylic acid esters, polyester ether copolymers, and organosilane terpolymers, and starch based emulsions, in particular organosilane terpolymers.

7. The textile material of any one of claims 5 or 6, wherein one or more chemical agents are fixed to the textile material which increase the antimicrobial efficacy of the textile material, selected from the group consisting of quaternary ammonium organosilane compounds, silver cations, polyglucosamine, propiconazole, carbendazim, isothiazoline, and polyhexamethylene biguanide, in particular of silver cations and propiconazole.

8. The textile material of any one of claims 5 to 7, wherein the stain release capability, antimicrobial efficiency, water holding capacity and/or water absorption time are achieved even after at least 25 laundry washes in a laundry washing machine at 85±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

9. The textile material of any one of claims 5 to 8, wherein the textile material is obtainable by the process of any one of claims 1 to 5.

10. A sanitary napkin comprising an inner layer which lies against the skin of the user when the sanitary napkin is worn by the user, the inner layer comprising or consisting of a textile material,
one or more absorption layers, each absorption layer comprising a textile material as defined in any one of claims 6 to 10, and
a water repellent layer, preferably having a water repellency rating of at least 70, preferably at least 80, more preferably at least 90, measured in accordance with AATCC test method 22-2014,
wherein the one or more absorption layers are located between the inner layer and the water repellent layer.

11. The sanitary napkin of claim 10, wherein the textile material of the inner layer has a water holding capacity of at most 2.0 times its weight, preferably of at most 1.0 times its weight, more preferably at most 0.5 times its weight, most preferably at most 0.2 times its weight, preferably when tested according to ASTM D7367 - 14, and/or
a water absorbency time of at least 2 minutes, preferably at least 3 minutes, measured in accordance with AATCC test method 79-2014 (Option A).

12. The sanitary napkin of any one of claims 10 or 11, wherein the textile material of the inner layer has a stain release capability, measured in accordance with AATCC test method 130-2010, of at least rating 3, preferably at least rating 4, and most preferably rating 5, and/or
wherein the textile material of the inner layer exhibits a reduction value of *Escherichia coli* ATCC 25922 and/or *Staphylococcus aureus* ATCC 6538 and/or *Pseudomonas aeruginosa* ATCC 15442 and/or *Salmonella enterica* ATCC 10708 and/or *Candida albicans* and/or *Aspergillus Niger,* measured in accordance with AATCC test method 100-2012 and/or ASTM E2149 - 10, of at least 90% (1 log), preferably at least 99% (2 log), more preferably at least 99.9% (3 log), within 1 hour of contact time, preferably remaining at the same level or greater even after 8 hours, more preferably increasing to at least 99.99% (4 log) within 8 hours.

13. The sanitary napkin of any one of claims 10 to 12, wherein the water repellent layer comprises or consists of a textile material, the textile material having a stain release capability, measured in accordance with AATCC test method 130-2010, of at least rating 3, preferably at least rating 4, and most preferably rating 5, and/or
a reduction value of *Escherichia coli* ATCC 25922 and/or *Staphylococcus aureus* ATCC 6538 and/or *Pseudomonas aeruginosa* ATCC 15442 and/or *Salmonella enterica* ATCC 10708 and/or *Candida albicans* and/or *Aspergillus Niger,* measured in accordance with AATCC test method 100-2012 and/or ASTM E2149 -10, of at least 90% (1 log), preferably remaining at the same level or greater even after 8 hours, more preferably increasing to at least 99% (2 log) within 8 hours.

14. The sanitary napkin of any one of claims 10 to 13, comprising a transportation layer comprising a textile material as defined in any one of claims 6 to 10, wherein the transportation layer is located between the inner layer and the one or more main absorption layers, and wherein the textile material is preferably an impression fabric, warp knitted, and/or a multifilament.

## Patentansprüche

1. Verfahren zum Veredeln eines textilen Faserstoffs, das einen antimikrobiellen Verfahrenszyklus und einen Hauptverfahrenszyklus umfasst, wobei der antimikrobielle Verfahrenszyklus vor dem Hauptverfahrenszyklus durchgeführt wird;
wobei der antimikrobielle Verfahrenszyklus die folgenden Schritte umfasst:
- Behandeln des textilen Faserstoffs unter Verwendung eines Auszehrverfahrens, wobei die Flotte ein oder mehrere chemische Mittel umfasst, die die antimikrobielle Wirksamkeit des textilen Faserstoffs erhöhen;
- Unterziehen des behandelten textilen Faserstoffs einer Wärmebehandlung,
und wobei der Hauptverfahrenszyklus die folgenden Schritte umfasst:
- Behandeln des textilen Faserstoffs unter Verwendung eines Auftragverfahrens wie etwa eines Rakel- oder Siebbeschichtungsverfahrens, eines Sprühverfahrens, eines Auszehrverfahrens oder bevorzugt eines Foulardierverfahrens, wobei eine Flotte auf die Textilie aufgetragen wird, die ein oder mehrere chemische Mittel umfasst, die das Schmutzabweisungsvermögen und das Wasserhaltevermögen des textilen Faserstoffs erhöhen, wobei die chemischen Mittel, die das Schmutzabweisungsvermögen und das Wasserhaltevermögen des textilen Faserstoffs erhöhen, auf wenigstens einem basieren, das aus der Gruppe ausgewählt ist, die aus Carbonsäureestern, Organosilanterpolymeren und einer Emulsion auf Stärkebasis besteht, und
- Unterziehen des behandelten textilen Faserstoffs einer Wärmebehandlung.

2. Verfahren nach Anspruch 1, wobei ein oder mehrere chemische Mittel, die die antimikrobielle Wirksamkeit des textilen Faserstoffs erhöhen, aus der Gruppe ausgewählt sind, die aus quaternären Ammoniumorganosilanverbindungen, Silberkationen, Polyglucosamin, Propiconazol und Polyhexamethylenbiguanid, insbesondere aus Silberkationen, Propiconazol und/oder Carbendazim und Isothiazolin besteht.

3. Verfahren nach einem der Ansprüche 1 bis 2, wobei die Wärmebehandlung des antimikrobiellen Verfahrenszyklus ein Trocknen umfasst, das wenigstens teilweise bei einer Temperatur von wenigstens 70 °C, bevorzugt wenigstens 100 °C, stärker bevorzugt wenigstens 100 °C, am stärksten bevorzugt wenigstens etwa 120 °C und bei einer Temperatur von höchstens 160 °C, bevorzugt von höchstens 140 °C, stärker bevorzugt von höchstens 130 °C und am stärksten bevorzugt von höchstens etwa 120 °C ausgeführt wird.

4. Verfahren nach einem der Ansprüche 1 bis 3, wobei die Wärmebehandlung des Hauptverfahrenszyklus ein Aushärten des textilen Faserstoffs umfasst, wobei das Aushärten wenigstens teilweise bei einer Temperatur von wenigstens 160 °C, bevorzugt wenigstens 170 °C, stärker bevorzugt wenigstens 175 °C und am stärksten bevorzugt wenigstens etwa 180 °C und bei einer Temperatur von höchstens 200 °C, bevorzugt höchstens 190 °C, stärker bevorzugt höchstens 185 °C und am stärksten bevorzugt höchstens 180 °C ausgeführt wird.

5. Textiler Faserstoff, der ein Schmutzabweisungsvermögen, das gemäß der AATCC-Prüfmethode 130-2010 gemessen wird, von wenigstens Bewertung 3, bevorzugt wenigstens Bewertung 4 und am stärksten bevorzugt Bewertung 5 aufweist,
wobei der textile Faserstoff einen Reduktionswert von *Escherichia coli* ATCC 25922 und/oder *Staphylococcus aureus* ATCC 6538 und/oder *Pseudomonas aeruginosa* ATCC 15442 und/oder *Salmonella enterica* ATCC 10708 und/oder *Candida albicans* und/oder *Aspergillus Niger,* der gemäß der AATCC-Prüfmethode 100-2012 und/oder ASTM E2149-10 gemessen wird, von wenigstens 90 % (1 log-Stufe), bevorzugt wenigstens 99 % (2 log-Stufen), stärker bevorzugt wenigstens 99,9 % (3 log-Stufen) innerhalb von 1 Stunde einer Kontaktzeit zeigt, der bevorzugt sogar nach 8 Stunden auf dem gleichen Niveau oder höher bleibt, der sich stärker bevorzugt innerhalb von 8 Stunden auf wenigstens 99,99 % (4 log-Stufen) erhöht, und
wobei der textile Faserstoff ein Wasserhaltevermögen von wenigstens dem 3-Fachen seines Gewichts, bevorzugt wenigstens dem 5-Fachen seines Gewichts, stärker bevorzugt wenigstens dem 7-Fachen seines Gewichts, insbesondere wenigstens dem 8-Fachen seines Gewichts, am stärksten bevorzugt wenigstens dem 12-Fachen seines Gewichts aufweist, bevorzugt wenn gemäß ASTM D7367-14 geprüft, und/oder wobei der textile Faserstoff eine Wasserabsorptionszeit von höchstens 5 Sekunden, bevorzugt höchstens 3 Sekunden, stärker bevorzugt höchstens 2 Sekunden und am stärksten bevorzugt höchstens 1 Sekunde aufweist, die gemäß der AATCC-Prüfmethode 79-2014 (Option A) gemessen wird.

6. Textiler Faserstoff nach Anspruch 5, wobei ein oder mehrere chemische Mittel an dem textilen Faserstoff fixiert sind, die das Schmutzabweisungsvermögen des textilen Faserstoffs erhöhen, wobei das eine oder die mehreren chemischen Mittel auf wenigstens einem basieren, das aus der Gruppe ausgewählt ist, die aus Carbonsäureestern, Polyester-Ether-Copolymeren und Organosilanterpolymeren und Emulsionen auf Stärkebasis, insbesondere Organosilanterpolymeren besteht.

7. Textiler Faserstoff nach einem der Ansprüche 5 oder 6, wobei ein oder mehrere chemische Mittel an dem textilen Faserstoff fixiert sind, die die antimikrobielle Wirksamkeit des textilen Faserstoffs erhöhen, die aus der Gruppe ausgewählt sind, die aus quaternären Ammoniumorganosilanverbindungen, Silberkationen, Polyglucosamin, Propiconazol, Carbendazim, Isothiazolin und Polyhexamethylenbiguanid, insbesondere aus Silberkationen und Propiconazol besteht.

8. Textiler Faserstoff nach einem der Ansprüche 5 bis 7, wobei das Schmutzabweisungsvermögen, die antimikrobielle Wirksamkeit, das Wasserhaltevermögen und/oder die Wasserabsorptionszeit sogar nach wenigstens 25 Wäschewaschungen in einer Wäschewaschmaschine 10-15 Minuten lang bei 85±15 °C, bevorzugt unter Verwendung von Tergitol 15-S-9 von Dow Chemicals, einem nicht antimikrobiellen, nichtionischen und nicht chlorhaltigen Waschmittel, bevorzugt gefolgt von einem Standardspülzyklus und bevorzugt 20-30 Minuten lang bei 62-96 °C getrocknet, erreicht wird.

9. Textiler Faserstoff nach einem der Ansprüche 5 bis 8, wobei der textile Faserstoff durch das Verfahren nach einem der Ansprüche 1 bis 5 erhaltbar ist.

10. Binde, die eine innere Schicht umfasst, die an die Haut des Benutzers anliegt, wenn die Binde von dem Benutzer getragen wird, wobei die innere Schicht einen textilen Faserstoff umfasst oder daraus besteht;
eine oder mehrere Absorptionsschichten, wobei jede Absorptionsschicht einen textilen Faserstoff nach einem der Ansprüche 6 bis 10 umfasst, und
eine wasserabstoßende Schicht, die bevorzugt eine Wasserabstoßungsbewertung von wenigstens 70, bevorzugt wenigstens 80, stärker bevorzugt wenigstens 90 aufweist, die gemäß der AATCC-Prüfmethode 22-2014 gemessen wird,
wobei sich die eine oder die mehreren Absorptionsschichten zwischen der inneren Schicht und der wasserabstoßenden Schicht befinden.

11. Binde nach Anspruch 10, wobei der textile Faserstoff der inneren Schicht ein Wasserhaltevermögen von höchstens dem 2,0-Fachen seines Gewichts, bevorzugt höchstens dem 1,0-Fachen seines Gewichts, stärker bevorzugt höchstens dem 0,5-Fachen seines Gewichts, am stärksten bevorzugt höchstens dem 0,2-Fachen seines Gewichts, bevorzugt wenn gemäß ASTM D7367-14 geprüft, und/oder
eine Wasserabsorptionszeit von wenigstens 2 Minuten, bevorzugt wenigstens 3 Minuten aufweist, die gemäß AATCC-Prüfmethode 79-2014 (Option A) gemessen wird.

12. Binde nach einem der Ansprüche 10 oder 11, wobei der textile Faserstoff der inneren Schicht ein Schmutzabweisungsvermögen, das gemäß der AATCC-Prüfmethode 130-2010 gemessen wird, von wenigstens Bewertung 3, bevorzugt wenigstens Bewertung 4 und am stärksten bevorzugt Bewertung 5 aufweist, und/oder
wobei der textile Faserstoff der inneren Schicht einen Reduktionswert von *Escherichia coli* ATCC 25922 und/oder *Staphylococcus aureus* ATCC 6538 und/oder *Pseudomonas aeruginosa* ATCC 15442 und/oder *Salmonella enterica* ATCC 10708 und/oder *Candida albicans* und/oder *Aspergillus Niger,* der gemäß der AATCC-Prüfmethode 100-2012 und/oder ASTM E2149-10 gemessen wird, von wenigstens 90 % (1 log-Stufe), bevorzugt wenigstens 99 % (2 log-Stufen), stärker bevorzugt wenigstens 99,9 % (3 log-Stufen) innerhalb von 1 Stunde der Kontaktzeit zeigt, der bevorzugt sogar nach 8 Stunden auf dem gleichen Niveau oder höher bleibt, der sich stärker bevorzugt innerhalb von 8 Stunden auf wenigstens 99,99 % (4 log-Stufen) erhöht.

13. Binde nach einem der Ansprüche 10 bis 12, wobei die wasserabstoßende Schicht einen textilen Faserstoff umfasst oder daraus besteht, wobei der textile Faserstoff ein Schmutzabweisungsvermögen, das gemäß der AATCC-Prüfmethode 130-2010 gemessen wird, von wenigstens Bewertung 3, bevorzugt wenigstens Bewertung 4 und am stärksten bevorzugt Bewertung 5, und/oder
einen Reduktionswert von *Escherichia coli ATCC* 25922 und/oder *Staphylococcus aureus* ATCC 6538 und/oder *Pseudomonas aeruginosa* ATCC 15442 und/oder *Salmonella enterica* ATCC 10708 und/oder *Candida albicans* und/oder *Aspergillus Niger,* der gemäß der AATCC-Prüfmethode 100-2012 und/oder ASTM E2149-10 gemessen wird, von wenigstens 90 % (1 log-Stufe) aufweist, der bevorzugt sogar nach 8 Stunden auf dem gleichen Niveau oder höher bleibt und stärker bevorzugt sich innerhalb von 8 Stunden auf wenigstens 99 % (2 log-Stufen) erhöht.

14. Binde nach einem der Ansprüche 10 bis 13, die eine Transportschicht umfasst, die einen textilen Faserstoff nach einem der Ansprüche 6 bis 10 umfasst, wobei sich die Transportschicht zwischen der inneren Schicht und der einen oder den mehreren Hauptabsorptionsschichten befindet; und wobei der textile Faserstoff bevorzugt ein Pressgewebe, kettengewirkt und/oder ein Multifilament ist.

## Revendications

1. Procédé de finition d'un matériau textile comprenant un cycle de traitement antimicrobien et un cycle de traitement principal, le cycle de traitement antimicrobien étant exécuté avant le cycle de traitement principal,
le cycle de traitement antimicrobien comprenant les étapes consistant à :
- traiter le matériau textile à l'aide d'un procédé d'épuisement, la liqueur comprenant un ou plusieurs agents chimiques qui augmentent l'efficacité antimicrobienne de la matière textile,
- soumettre le matériau textile traité à un traitement thermique,
et le cycle de traitement principal comprenant les étapes consistant à :
- traiter le matériau textile à l'aide d'un processus d'application tel qu'un processus d'enduction à la racle ou au cadre, un processus de pulvérisation, un processus d'épuisement ou de préférence un processus de foulardage, une lessive étant appliquée au textile qui comprend un ou plusieurs agents chimiques qui augmentent la capacité d'aptitude au détachage et la capacité de rétention d'eau du matériau textile, les agents chimiques qui augmentent la capacité d'aptitude au détachage et la capacité de rétention d'eau du matériau textile étant à base d'au moins un élément choisi dans le groupe constitué par les esters d'acide carboxylique, les terpolymères d'organosilane et une émulsion à base d'amidon, et
- soumettre le matériau textile traité à un traitement thermique.

2. Procédé selon la revendication 1, un ou plusieurs agents chimiques qui augmentent l'efficacité antimicrobienne du matériau textile étant choisis dans le groupe constitué par les composés organosilane d'ammonium quaternaire, les cations d'argent, la polyglucosamine, le propiconazole et le polyhexaméthylène biguanide, en particulier les cations d'argent, le propiconazole et/ou le carbendazime et l'isothiazoline.

3. Procédé selon l'une quelconque des revendications 1 à 2, le traitement thermique du cycle de traitement antimicrobien comprenant un séchage effectué au moins partiellement à une température d'au moins 70 °C, de préférence d'au moins 100 °C, plus préférablement d'au moins 100 °C, le plus préférablement au moins environ 120 °C et à une température d'au plus 160 °C, de préférence d'au plus 140 °C, plus préférablement d'au plus 130 °C, et le plus préférablement d'au plus environ 120 °C.

4. Procédé selon l'une quelconque des revendications 1 à 3, le traitement thermique du cycle de traitement principal comprenant le durcissement du matériau textile, le durcissement ayant lieu au moins partiellement à une température d'au moins 160 °C, de préférence d'au moins 170 °C, plus préférablement d'au moins 175 °C, et le plus préférablement d'au moins environ 180 °C, et à une température d'au plus 200 °C, de préférence au plus 190 °C, plus préférablement au plus 185 °C et le plus préférablement 180 °C.

5. Matériau textile présentant une capacité d'aptitude au détachage, mesurée selon le procédé d'essai AATCC 130-2010, d'au moins niveau 3, de préférence d'au moins niveau 4 et le plus préférablement de niveau 5,
le matériau textile présentant une valeur de réduction *d'Escherichia coli ATCC* 25922 et/ou de *Staphylococcus aureus* ATCC 6538 et/ou de *Pseudomonas aeruginosa* ATCC 15442 et/ou de *Salmonella enterica* ATCC 10708 et/ou de *Candida albicans* et/ou *d'Aspergillus Niger,* mesurée conformément au procédé d'essai AATCC 100-2012 et/ou ASTM E2149-10, d'au moins 90 % (1 log), de préférence d'au moins 99 % (2 log), plus préférablement d'au moins 99,9 % (3 log), dans l'heure du temps de contact, de préférence restant en la même concentration ou supérieure même après 8 heures, plus préférablement augmentant à au moins 99,99 % (4 log) dans les 8 heures, et
le matériau textile ayant une capacité de rétention d'eau d'au moins 3 fois son poids, de préférence d'au moins 5 fois son poids, plus préférablement d'au moins 7 fois son poids, en particulier d'au moins 8 fois son poids, le plus préférablement d'au moins 12 fois son poids, de préférence lors d'un essai selon ASTM D7367-14, et/ou le matériau textile ayant un temps d'absorption d'eau d'au plus 5 secondes, de préférence d'au plus 3 secondes, plus préférablement d'au plus 2 secondes et le plus préférablement d'au plus 1 seconde, mesuré conformément au procédé d'essai AATCC 79-2014 (option A).

6. Matériau textile selon la revendication 5, un ou plusieurs agents chimiques étant fixés au matériau textile, ce qui augmente la capacité d'aptitude au détachage du matériau textile, l'un ou les agents chimiques étant à base d'au moins un agent choisi dans le groupe constitué par les esters d'acide carboxylique, les copolymères d'éther de polyester et les terpolymères d'organosilane, et les émulsions à base d'amidon, en particulier les terpolymères d'organosilane.

7. Matériau textile selon l'une quelconque des revendications 5 ou 6, un ou plusieurs agents chimiques étant fixés au matériau textile, ce qui augmente l'efficacité antimicrobienne du matériau textile, choisi dans le groupe constitué par les composés organosilane d'ammonium quaternaire, les cations d'argent, la polyglucosamine, le propiconazole, le carbendazime, l'isothiazoline et le polyhexaméthylène biguanide, en particulier les cations d'argent et le propiconazole.

8. Matériau textile selon l'une quelconque des revendications 5 à 7, la capacité d'aptitude au détachage, l'efficacité antimicrobienne, la capacité de rétention d'eau et/ou le temps d'absorption d'eau étant obtenus même après au moins 25 lavages dans une machine à laver à 85 ± 15 °C pendant 10 à 15 minutes, de préférence avec du Tergitol 15-S-9 de Dow Chemicals, un détergent à lessive non antimicrobien, non ionique et ne contenant pas de chlore, de préférence suivi d'un cycle de rinçage standard et de préférence séché à une température de 62 à 96 °C pendant 20 à 30 minutes.

9. Matériau textile selon l'une quelconque des revendications 5 à 8, le matériau textile pouvant être obtenu par le procédé de l'une quelconque des revendications 1 à 5.

10. Serviette hygiénique comprenant une couche intérieure qui repose contre la peau de l'utilisateur lorsque la serviette hygiénique est portée par l'utilisateur, la couche intérieure comprenant ou étant constituée d'un matériau textile,
une ou plusieurs couches d'absorption, chaque couche d'absorption comprenant un matériau textile tel que défini dans l'une quelconque des revendications 6 à 10, et
une couche hydrophobe, ayant de préférence un indice d'hydrophobie d'au moins 70, de préférence d'au moins 80, plus préférablement d'au moins 90, mesuré selon le procédé d'essai AATCC 22-2014,
l'une ou les couches d'absorption étant situées entre la couche interne et la couche hydrophobe.

11. Serviette hygiénique selon la revendication 10, le matériau textile de la couche intérieure ayant une capacité de rétention d'eau d'au plus 2,0 fois son poids, de préférence d'au plus 1,0 fois son poids, plus préférablement d'au plus 0,5 fois son poids, plus préférablement d'au plus 0,2 fois son poids, de préférence lors d'essais selon la norme ASTM D7367-14, et/ou un temps d'absorption d'eau d'au moins 2 minutes, de préférence d'au moins 3 minutes, mesuré selon le procédé d'essai AATCC 79-2014 (option A).

12. Serviette hygiénique selon l'une quelconque des revendications 10 ou 11, le matériau textile de la couche intérieure ayant une capacité d'aptitude au détachage, mesurée selon le procédé d'essai AATCC 130-2010, d'au moins indice 3, de préférence d'au moins indice 4, et le plus préférablement d'indice 5, et/ou
le matériau textile de la couche intérieure présentant une valeur de réduction *d'Escherichia coli ATCC* 25922 et/ou de *Staphylococcus aureus* ATCC 6538 et/ou de *Pseudomonas aeruginosa* ATCC 15442 et/ou de *Salmonella enterica* ATCC 10708 et/ou de *Candida albicans* et/ou *d'Aspergillus Niger,* mesurée conformément au procédé d'essai AATCC 100-2012 et/ou ASTM E2149-10, d'au moins 90 % (1 log), de préférence d'au moins 99 % (2 log), plus préférablement d'au moins 99,9 % (3 log), dans l'heure du temps de contact, de préférence restant en la même concentration ou supérieure même après 8 heures, plus préférablement augmentant à au moins 99,99 % (4 log) dans les 8 heures.

13. Serviette hygiénique selon l'une quelconque des revendications 10 à 12, la couche hydrophobe comprenant ou étant constituée d'un matériau textile, le matériau textile ayant une capacité d'aptitude au détachage, mesurée selon le procédé d'essai AATCC 130-2010, d'au moins indice 3, de préférence au moins indice 4, et plus préférablement d'indice 5, et/ou
une valeur de réduction *d'Escherichia coli ATCC* 25922 et/ou de *Staphylococcus aureus* ATCC 6538 et/ou de *Pseudomonas aeruginosa* ATCC 15442 et/ou de *Salmonella enterica* ATCC 10708 et/ou de *Candida albicans* et/ou *d'Aspergillus Niger,* mesurée conformément au procédé d'essai AATCC 100-2012 et/ou ASTM E2149-10, d'au moins 90 % (1 log), de préférence restant au même niveau ou supérieur même après 8 heures, plus préférablement augmentant à au moins 99 % (2 log) dans les 8 heures.

14. Serviette hygiénique selon l'une quelconque des revendications 10 à 13, comprenant une couche de transport comprenant un matériau textile tel que défini dans l'une quelconque des revendications 6 à 10, la couche de transport étant située entre la couche intérieure et l'au moins une absorption principale de couches, et le matériau textile étant de préférence un tissu d'impression, un tricot en chaîne et/ou un multifilament.
